# EUROPEAN PATENT APPLICATION

(11) **EP 3 741 399 A1**
(43) Date of publication of application: **25.11.2020**
(21) Application number: 19840412.1
(22) Date of filing: 26.07.2019
(51) Int. Cl.: A61L 27/38, A61L 27/18, A61L 27/22, A61L 27/24, A61L 27/36, A61L 27/40, A61L 27/52, C12N 5/071, C12N 5/077

(54) **SHEET-LIKE CELL-CULTURED PRODUCT FOR REGENERATION OF GASTROINTESTINAL TRACT**

(30) Priority: 27.07.2018 JP 2018140977
(71) Applicant: NAGASAKI UNIVERSITY, Nagasaki 852-8521 (JP); Terumo Kabushiki Kaisha, Tokyo 151-0072 (JP)
(72) Inventor: MARUYA, Yasuhiro, Nagasaki-shi, Nagasaki 852-8521 (JP); MATSUMOTO, Ryo, Nagasaki-shi, Nagasaki 852-8521 (JP); KOBAYASHI, Shinichiro, Nagasaki-shi, Nagasaki 852-8521 (JP); KANETAKA, Kengo, Nagasaki-shi, Nagasaki 852-8521 (JP); EGUCHI, Susumu, Nagasaki-shi, Nagasaki 852-8521 (JP); OHNITA, Ken, Nagasaki-shi, Nagasaki 852-8521 (JP); HASHIGUCHI, Keiichi, Nagasaki-shi, Nagasaki 852-8521 (JP); OHASHI, Fumiya, Ashigarakami-gun, Kanagawa 259-0151 (JP); MATSUMURA, Masaki, Ashigarakami-gun, Kanagawa 259-0151 (JP)
(74) Representative: Prüfer & Partner mbB Patentanwälte · Rechtsanwälte
(86) International application number: PCT/JP2019/029479
(87) International publication number: WO 2020/022494

(57) **Abstract**

In the present specification, a sheet-shaped cell culture for promoting healing of a lumen organ having a damaged part, particularly tissue of a duodenum; a method for producing the sheet-shaped cell culture; a method for regenerating a damaged part in a lumen organ using the sheet-shaped cell culture; and the like are provided.

## Description

### Technical Field

The present invention relates to a sheet-shaped cell culture for treating a lumen organ having a damaged part, a method for producing the sheet-shaped cell culture, a method for treating a lumen organ using the sheet-shaped cell culture, and the like.

### Background Art

In recent years, attempts have been made to transplant various cells to repair damaged tissue and the like. For example, use of fetal cardiomyocytes, skeletal muscle myoblast cells, mesenchymal stem cells, cardiac stem cells, ES cells, iPS cells, and the like has been attempted to repair damaged cardiac muscle tissue caused by ischemic heart diseases such as angina pectoris and myocardial infarction (NPL 1).

As part of such attempts, cell structures formed using scaffolds, and sheet-shaped cell cultures formed by forming cells into a sheet shape have been developed (PTL 1 and NPL 2) .

For therapeutic application of sheet-shaped cell cultures, there are ongoing studies on use of a cultured epidermal sheet for skin damage due to a burn injury or the like, use of a corneal epithelial sheet-shaped cell culture for corneal damage, use of an oral mucosal sheet-shaped cell culture for endoscopic resection of esophageal cancer, and the like, some of which are in the stage of clinical applications.

As one of such applications, it has been proposed to use a sheet-shaped cell culture for healing damage of organs such as a digestive tract. For example, PTL 2 discloses use of a sheet-shaped cell culture containing mesenchymal stem cells to heal or prevent leakage from a damaged part of a digestive tract caused by suturing failure or the like. Furthermore, NPL 3 and NPL 4 disclose that a sheet of skeletal muscle myoblast cells can be used for healing of pancreatic fistula or gastric perforation in an animal model.

In recent years, endoscopic submucosal dissection (ESD) has been attracting attention as a method of resecting an early-stage tumor in a less invasive manner. This involves injecting a local injection into a submucosal layer of a lesion area, artificially causing edema, and then resecting a raised mucosa lesion area together with the submucosal layer. However, it has been reported that ESD of particularly a duodenum causes complications such as perforation with a probability of about 30% due to operability of an endoscope and thinness of an intestinal wall.

### Citation List

### Patent Literature

PTL 1: JP-T-2007-528755
PTL 2: International Publication No. 2017/130802

### Non Patent Literature

NPL 1: Haraguchi et al., Stem Cells Transl Med. 2012 Feb; 1(2): 136-41
NPL 2: Sawa et al., Surg Today. 2012 Jan; 42(2): 181-4
NPL 3: Tanaka et al., J Gastroenterol. 2013 Sep; 48(9) : 1081-9
NPL 4: Tanaka et al., Surg Today. 2017 Jan; 47(1): 114-121

### Summary of Invention

### Technical Problem

An object of the present disclosure is to provide a sheet-shaped cell culture for regenerating a lumen organ having a damaged part, particularly tissue of a duodenum; a method for producing the sheet-shaped cell culture; a method for regenerating a damaged part in a lumen organ using the sheet-shaped cell culture; and the like.

### Solution to Problem

The inventors of the present invention have diligently studied a method of reducing postoperative complications of ESD of a duodenum, and have found for the first time that it is possible to maintain continuity of a ulcer base and prevent postoperative perforation by attaching a sheet-shaped cell culture of skeletal muscle myoblast cells to an outer side of an ESD procedure site. As a result of further study based on such findings, they have completed the prevent invention.

That is, the present invention relates to the following aspects.
[1] A sheet-shaped cell culture for promoting healing of a lumen organ having a damaged part on at least one side of a lumen wall, in which the sheet-shaped cell culture is applied to a corresponding opposite site of the lumen wall of a site in which damage is present.
[2] The sheet-shaped cell culture according to [1], in which the damage is on an inner portion of the lumen wall, and the sheet-shaped cell culture is applied to a corresponding outer portion of the lumen wall, or the damage is on an outer portion of the lumen wall, and the sheet-shaped cell culture is applied to a corresponding inner portion of the lumen wall.
[3] The sheet-shaped cell culture according to [1], in which the damage is penetrating damage or non-penetrating damage.
[4] The sheet-shaped cell culture according to any one of [1] to [3], in which the lumen organ is an organ of a digestive system.
[5] The sheet-shaped cell culture according to any one of [1] to [4], in which the damage is caused by dissection of surface tissue of the lumen wall.
[6] The sheet-shaped cell culture according to any one of [1] to [5], including a reinforcement layer containing a gel and/or a polymer.
[7] The sheet-shaped cell culture according to any one of [1] to [6], in which the sheet-shaped cell culture is applied together with a pedunculated blood vessel.
[8] The sheet-shaped cell culture according to any one of [1] to [7], in which the sheet-shaped cell culture has a thickness of 50 to 500 µm.
[9] The sheet-shaped cell culture according to any one of [1] to [8], in which the sheet-shaped cell culture contains a cell population having a seeding density of about 7.1 × 10⁵ cells/cm² to about 3.0 × 10⁶ cells/cm².
[10] The sheet-shaped cell culture according to any one of [1] to [9], in which the sheet-shaped cell culture is for preventing inflammation occurring due to a surgical treatment and/or inflammation occurring due to perforation caused by a surgical treatment.
[11] A method for preventing perforation in a lumen organ after a surgical treatment, the method including a step of applying a sheet-shaped cell culture to the lumen organ, in which the sheet-shaped cell culture is applied to a treated site or a corresponding opposite site of the treated site.
[12] The method according to [11], in which the surgical treatment is dissection of a mucosa, a muscularis mucosa, and/or a submucosal layer of the lumen organ.
[13] The method according to [11] or [12], in which the sheet-shaped cell culture is applied such that the sheet-shaped cell culture entirely covers the treated site or the corresponding opposite site of the treated site.
[14] The method according to any one of [11] to [13], in which the sheet-shaped cell culture is delivered to the treated site or the corresponding opposite site of the treated site by a delivery device.
[15] A method for treating a diseased part on a surface of a lumen wall, the method including:
   step (a) of dissecting the diseased part on the surface of the lumen wall; and
   step (b) of applying the sheet-shaped cell culture to a dissected portion obtained in step (a) or a corresponding opposite portion of the dissected portion.
[16] The method according to [15], in which a disease is cancer.
[17] The method according to [15] or [16], in which the dissection of the diseased part on the surface of the lumen wall is dissection of a mucosa, a muscularis mucosa, and/or a submucosal layer having the diseased part.

### Advantageous Effects of Invention

According to the present invention, it is possible to promote healing of lumen tissue having damage on at least one side of a lumen wall by transplanting a sheet-shaped cell culture into a corresponding opposite site of a site in which the damage is present. The sheet-shaped cell culture is particularly effective even for damage formed due to procedures such as ESD, and thus enables prevention of complications after the procedures and improvement in prognosis.

### Brief Description of Drawings

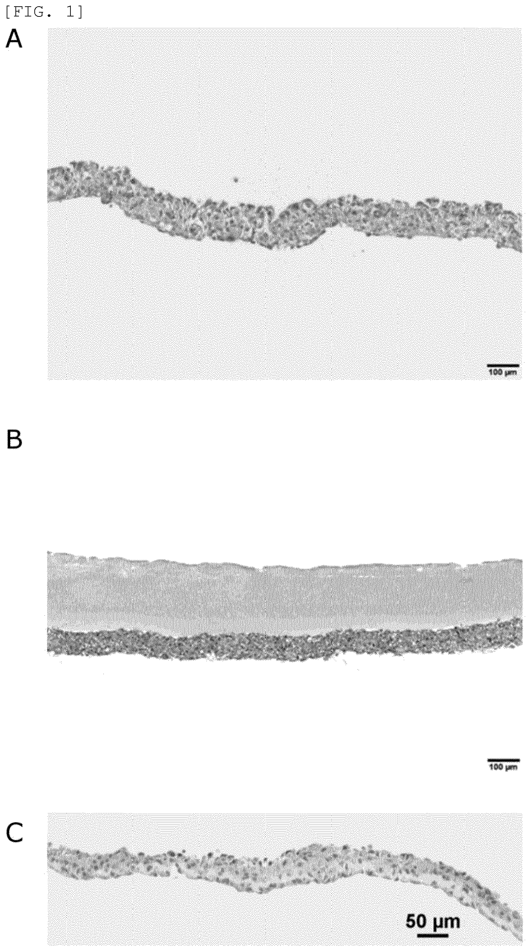

[FIG. 1] FIG. 1 shows histological stained images of sheet-shaped cell cultures of the present disclosure. FIG. 1A is a stained image of a sheet-shaped cell culture of porcine skeletal muscle myoblast cells, FIG. 1B is a stained image of a sheet-shaped cell culture of porcine skeletal muscle myoblast cells in which a reinforcement layer formed of fibrin gel is formed, and FIG. 1C is a stained image of a sheet-shaped cell culture of porcine mesenchymal stem cells. A sheet thickness of the sheet-shaped cell culture of skeletal muscle myoblast cells was about 100 µm, whereas a sheet thickness of the sheet-shaped cell culture of porcine mesenchymal stem cells was less than 50 µm.

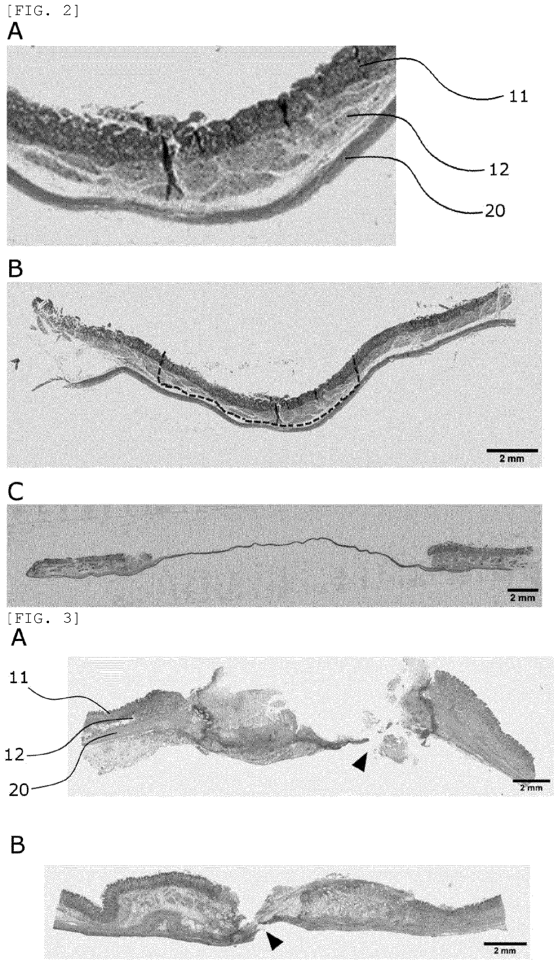

[FIG. 2] FIG. 2 shows histological stained images of a digestive tract wall of a duodenum. FIG. 2A is a cross-sectional stained image showing a structure of a porcine duodenum wall. 11 denotes a mucosa, 12 denotes a submucosal layer (Brunner's gland), and 20 denotes a muscular layer. FIG. 2B is a histological stained image before ESD treatment, and a dotted line portion is resected. FIG. 2C is a stained image after the ESD treatment. It can be seen that the mucosa and the submucosal layer were removed, and the muscular layer remained.
[FIG. 3] FIG. 3 shows histological stained images of ESD procedure sites of a pig as a control group. FIG. 3A represents a target group 1, and FIG. 3B represents a target group 2. It can be seen that at any of the procedure sites, continuity of a ulcer base disappeared at a site indicated by an arrowhead, and postoperative perforation occurred.

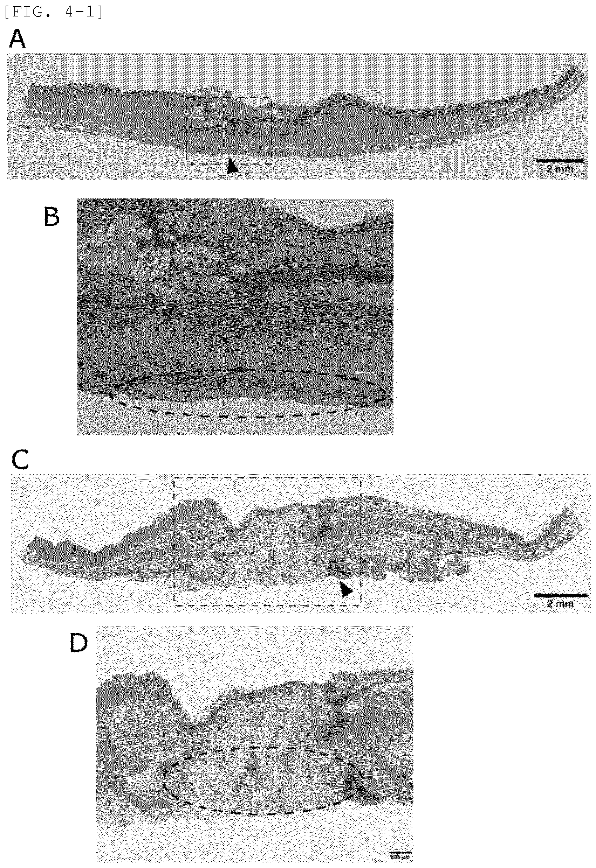

[FIG. 4-1] FIG. 4-1 shows histological stained images of ESD procedure sites of a pig in a sheet treatment group. FIG. 4-1A and FIG. 4-1B represent a sheet treatment group 1, FIG. 4-1C and FIG. 4-1D represent a sheet treatment group 2, FIG. 4-1E and FIG. 4-1F represent a sheet treatment group 3, and FIG. 4-1G and FIG. 4-1H represent a sheet treatment group 4. Sites indicated by arrowheads in FIG. 4-1A, FIG. 4-1C, and FIG. 4-1F are sites to which the sheet-shaped cell culture of porcine skeletal muscle myoblast cells was attached, and it can be seen that continuity of a ulcer base was maintained, and postoperative perforation was histologically prevented. In addition, FIG. 4-1B and FIG. 4-1D are respectively enlarged views of sites surrounded by dotted line squares of FIG. 4-1A and FIG. 4-1C, and fibrin, which is a reinforcement layer of the attached sheet-shaped cell culture, can be observed at a site surrounded by a dotted line ellipse of FIG. 4-1B. Furthermore, it can be seen that continuity of fibrin disappeared at a site surrounded by a dotted line ellipse of FIG. 4-1D. Furthermore, fibrin, which is a reinforcement layer of the attached sheet-shaped cell culture, can be observed at a site indicated by an arrowhead of FIG. 4-1F.

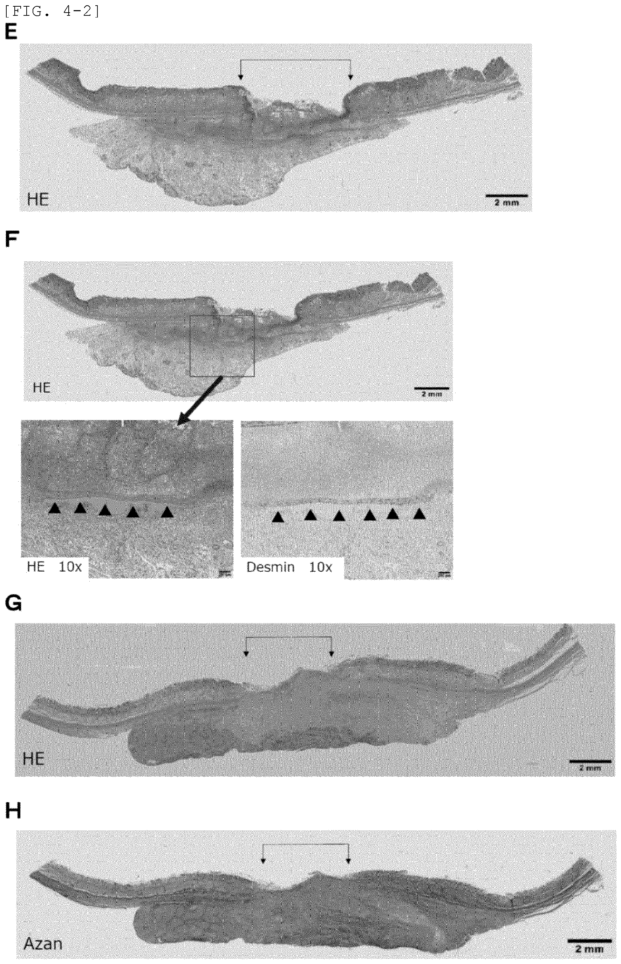

[FIG. 4-2] FIG. 4-2 shows histological stained images of ESD procedure sites of a pig in a sheet treatment group. FIG. 4-1A and FIG. 4-1B represent a sheet treatment group 1, FIG. 4-1C and FIG. 4-1D represent a sheet treatment group 2, FIG. 4-1E and FIG. 4-1F represent a sheet treatment group 3, and FIG. 4-1G and FIG. 4-1H represent a sheet treatment group 4. Sites indicated by arrowheads in FIG. 4-1A, FIG. 4-1C, and FIG. 4-1F are sites to which the sheet-shaped cell culture of porcine skeletal muscle myoblast cells was attached, and it can be seen that continuity of a ulcer base was maintained, and postoperative perforation was histologically prevented. In addition, FIG. 4-1B and FIG. 4-1D are respectively enlarged views of sites surrounded by dotted line squares of FIG. 4-1A and FIG. 4-1C, and fibrin, which is a reinforcement layer of the attached sheet-shaped cell culture, can be observed at a site surrounded by a dotted line ellipse of FIG. 4-1B. Furthermore, it can be seen that continuity of fibrin disappeared at a site surrounded by a dotted line ellipse of FIG. 4-1D. Furthermore, fibrin, which is a reinforcement layer of the attached sheet-shaped cell culture, can be observed at a site indicated by an arrowhead of FIG. 4-1F.

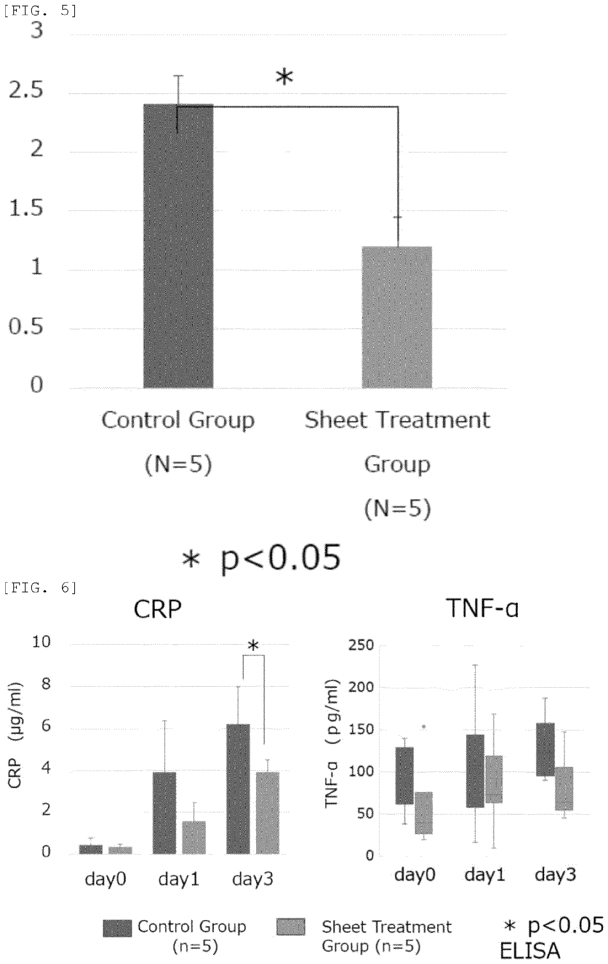

[FIG. 5] FIG. 5 shows data in which adhesion scores in Table 1 are graphed.
[FIG. 6] FIG. 6 shows transition of an inflammatory response. An increase or decrease in the inflammatory response was expressed as an increase or decrease in expression of C-reactive protein (CRP) and TNF-α in plasma.

### Description of Embodiments

Hereinafter, the present disclosure will be described in detail.

Unless defined otherwise in the present specification, all technical terms and scientific terms used in the present specification have the same meaning as terms commonly understood by those skilled in the art. All patents, applications, published applications, and other published materials referenced in the present specification are incorporated in the present specification by reference in their entirety. In addition, in a case where there is a contradiction between a published material referenced in the present specification and the description of the present specification, the description of the present specification takes priority.

In the present disclosure, the term "sheet-shaped cell culture" refers to a cell culture formed into a sheet shape by connecting cells to each other. Cells may be connected to each other directly (including a case in which cells are connected via a cell element such as an adhesion molecule) and/or via an intervening substance. The intervening substance is not particularly limited as long as it is a substance that can connect cells to each other at least physically (mechanically), and examples thereof include extracellular matrix and the like. The intervening substance is preferably derived from cells and is particularly preferably derived from cells constituting a cell culture. Cells are at least physically (mechanically) connected, but may be further connected functionally, for example chemically or electrically. The sheet-shaped cell culture may be composed of one cell layer (a single layer), and may be composed of two or more cell layers (a laminate (a multilayer) of, for example, two layers, three layers, four layers, five layers, six layers, and the like) . Furthermore, the sheet-shaped cell culture may have a three-dimensional structure which has a thickness exceeding a thickness of one cell and in which cells do not show a clearly layered structure. For example, in a vertical cross section of the sheet-shaped cell culture, cells may be present in a non-uniformly disposed state (for example, a mosaic-like state) without being uniformly aligned in a horizontal direction.

The sheet-shaped cell culture preferably does not contain a scaffold (support). A scaffold is used sometimes in the present technical field to allow cells to adhere onto its surface and/or to adhere inside thereof, thereby maintaining physical integrity of a sheet-shaped cell culture. For example, a film made of polyvinylidene difluoride (PVDF), and the like are known, but the sheet-shaped cell culture of the present disclosure can maintain its physical integrity without such scaffolds. Furthermore, it is preferable that the sheet-shaped cell culture of the present disclosure be composed of only a substance derived from cells constituting the sheet-shaped cell culture, and do not contain any other substance.

Cells constituting the sheet-shaped cell culture are not particularly limited as long as they can form the sheet-shaped cell culture, and examples thereof include adherent cells (adhesive cells) . Examples of adherent cells include adherent somatic cells (for example, cardiomyocytes, fibroblast cells, epithelial cells, endothelial cells, hepatocytes, pancreatic cells, renal cells, adrenal cells, periodontal ligament cells, gingival cells, periosteal cells, skin cells, synovial cells, chondrocytes, and the like), stem cells (tissue stem cells such as myoblast cells and cardiac stem cells, pluripotent stem cells such as embryonic stem cells and induced pluripotent stem (iPS) cells, mesenchymal stem cells, and the like), and the like. Somatic cells may be differentiated from stem cells, particularly from iPS cells (adherent cells derived from iPS cells). Non-limiting examples of cells constituting the sheet-shaped cell culture include myoblast cells (for example, skeletal muscle myoblast cells and the like), mesenchymal stem cells (for example, cells derived from bone marrow, adipose tissue, peripheral blood, skin, hair root, muscle tissue, endometrium, placenta, or cord blood, and the like), cardiomyocytes, fibroblast cells, cardiac stem cells, embryonic stem cells, iPS cells, synovial cells, chondrocytes, epithelial cells (for example, oral mucosal epithelial cells, retinal pigment epithelial cells, nasal mucosal epithelial cells, and the like), endothelial cells (for example, vascular endothelial cells and the like), hepatocytes (for example, hepatic parenchymal cells and the like), pancreatic cells (for example, pancreatic islet cells and the like), kidney cells, adrenal cells, periodontal ligament cells, gingival cells, periosteal cells, skin cells, and the like. Non-limiting examples of adherent cells derived from iPS cells include cardiomyocytes, fibroblast cells, epithelial cells, endothelial cells, hepatocytes, pancreatic cells, renal cells, adrenal cells, periodontal ligament cells, gingival cells, periosteal cells, skin cells, synovial cells, chondrocytes, and the like, all of which are derived from iPS cells.

In the present disclosure, a "skeletal muscle myoblast cell" means a myoblast cell present in skeletal muscle. Skeletal muscle myoblast cells are well known in the present technical field. They can be prepared from skeletal muscle by any known method (for example, a method described in JP-A-2007-89442, and the like), or it is possible to obtain commercially available products thereof (for example, Lonza, Cat# CC-2580) . Skeletal muscle myoblast cells are not limited, and they can be identified by, for example, markers such as CD56, α7 integrin, myosin heavy chain IIa, myosin heavy chain IIb, myosin heavy chain IId (IIx), MyoD, Myf5, Myf6, myogenin, desmin, and PAX3. In a specific aspect, skeletal muscle myoblast cells are CD56-positive. In a more specific aspect, skeletal muscle myoblast cells are CD56-positive and desmin-positive. Skeletal muscle myoblast cells are derived from any organisms having skeletal muscle without limitations. For example, they may be derived from humans, non-human primates, rodents (mice, rats, hamsters, guinea pigs, and the like), mammals such as rabbits, dogs, cats, pigs, horses, cows, goats, and sheep. In one aspect, skeletal muscle myoblast cells are skeletal muscle myoblast cells of mammals. In a specific aspect, skeletal muscle myoblast cells are skeletal muscle myoblast cells of human. Furthermore, skeletal muscle myoblast cells can be collected from any skeletal muscle. In one aspect, the skeletal muscle myoblast cells of the present disclosure are skeletal muscle myoblast cells derived from a femoral region, a neck region, or an abdominal region.

Cells constituting the sheet-shaped cell culture may be derived from any organism that can be treated with the sheet-shaped cell culture. Examples of such organisms include, but are not limited to, humans, non-human primates, dogs, cats, pigs, horses, goats, sheep, rodents (for example, mice, rats, hamsters, guinea pigs, and the like), rabbits, and the like. Furthermore, the number of types of cell constituting the sheet-shaped cell culture is not particularly limited, and the sheet-shaped cell culture may be constituted by only one type of cell, or by two or more types of cell. In a case where there are two or more types of cell forming the sheet-shaped cell culture, a content ratio (degree of purity) of the most abundant cells is 50% or more, preferably 60% or more, more preferably 70% or more, and even more preferably 75% or more at the point in which formation of the sheet-shaped cell culture is completed.

Cells may be xenogeneic cells or allogeneic cells. The term "xenogeneic cells" referred to herein means cells derived from an organism of a species different from that of a recipient in a case where the sheet-shaped cell culture is used for transplantation. For example, xenogeneic cells correspond to cells derived from monkeys or pigs and the like in a case where a recipient is human. In addition, the term "allogeneic cells" means cells derived from an organism of the same species as that of a recipient. For example, allogeneic cells correspond to human cells in a case where a recipient is human. Allogeneic cells include autologous cells (also called autologous cells or autologous cells), that is, cells derived from a recipient, and allogeneic non-autologous cells (also called allogeneic cells). Autologous cells are preferable in the present disclosure because they do not cause a rejection reaction when transplanted. However, it is also possible to use xenogeneic cells or allogeneic non-autologous cells. In a case where xenogeneic cells or allogeneic non-autologous cells are used, an immunosuppressive treatment may be required to suppress a rejection reaction. In the present specification, cells other than autologous cells, that is, xenogeneic cells and allogeneic non-autologous cells may be collectively referred to as non-autologous cells. In one aspect of the present disclosure, cells are autologous cells or allogeneic cells. In another aspect of the present disclosure, cells are autologous cells. In still another aspect of the present disclosure, cells are allogeneic cells.

The sheet-shaped cell culture can be produced by any known method (for example, refer to PTL 1, JP-A-2010-081829, JP-A-2011-110368, and the like). A method for producing a sheet-shaped cell culture typically includes a step of seeding cells on a culture substrate, a step of sheet-forming of the seeded cells, and a step of detaching the formed sheet-shaped cell culture from the culture substrate, but steps are not limited to these steps. A step of freezing cells and a step of thawing cells may be performed before the step of seeding cells on a culture substrate. Furthermore, a step of washing cells may be performed after the step of thawing cells. Each of these steps can be performed by any known method suitable for producing the sheet-shaped cell culture. The production method of the present disclosure may include a step of producing a sheet-shaped cell culture, and in this case, the step of producing a sheet-shaped cell culture may include one or two or more of steps relating the method for producing a sheet-shaped cell culture as sub-steps. In one aspect, a step of proliferating cells is not included before the step of seeding cells on a culture substrate and after the step of thawing cells.

The culture substrate is not particularly limited as long as cells can form a cell culture thereon, and examples thereof include containers of various materials, solid or semi-solid surfaces of the containers, and the like. A container preferably has a structure or material which does not allow liquids such as a culture solution to permeate. Examples of such materials include, but are not limited to, polyethylene, polypropylene, Teflon (registered trademark), polyethylene terephthalate, polymethyl methacrylate, nylon 6,6, polyvinyl alcohol, cellulose, silicon, polystyrene, glass, polyacrylamide, polydimethylacrylamide, metal (for example, iron, stainless steel, aluminum, copper, brass), and the like. In addition, the container preferably has at least one flat surface. Examples of such containers include, but are not limited to, a culture container having a bottom composed of a culture substrate on which a cell culture can be formed, and a liquid-impermeable side surface. Specific examples of such culture containers include, but are not limited to, cell culture dishes, cell culture bottles, and the like. The bottom of the container may be transparent or opaque. In a case where the bottom of the container is transparent, cells can be observed, counted, and the like from a back side of the container. Furthermore, the container may have a solid or semi-solid surface therein. Examples of solid surfaces include plates and containers made of various materials as described above, and the like, and examples of semi-solid surfaces include gels, soft polymer matrices, and the like. The culture substrate may be produced using the above-mentioned materials, or commercially available products may be used. Preferable examples of culture substrates include, but are not limited to, a substrate having an adhesive surface suitable for forming a sheet-shaped cell culture. Specific examples thereof include a substrate having a hydrophilic surface, for example, a substrate having a surface coated with hydrophilic compounds such as polystyrene subjected to a corona discharge treatment, collagen gels, and hydrophilic polymers; a substrate having a surface coated with extracellular matrices such as collagen, fibronectin, laminin, vitronectin, proteoglycan, and glycosaminoglycan, cell adhesion factors such as the cadherin family, the selectin family, and the integrin family, or the like; and the like. Furthermore, such substrates are commercially available (for example, Corning^{(R)} TC-Treated Culture Dish from Corning, and the like). The whole or part of the culture substrate may be transparent or opaque.

A surface of the culture substrate may be coated with a material having physical properties that change in response to stimuli such as temperature and light. As such materials, it is possible to use known materials such as a temperature responsive material consisting of a homopolymer or a copolymer of a (meth)acrylamide compound, N-alkyl substituted (meth)acrylamide derivatives (for example, N-ethylacrylamide, N-n-propylacrylamide, N-n-propylmethacrylamide, N-isopropylacrylamide, N-isopropylmethacrylamide, N-cyclopropylacrylamide, N-cyclopropylmethacrylamide, N-ethoxyethylacrylamide, N-ethoxyethylmethacrylamide, N-tetrahydrofurfurylacrylamide, N-tetrahydrofurfurylmethacrylamide, and), N,N-dialkyl substituted (meth)acrylamide derivatives (for example, N,N-dimethyl(meth)acrylamide, N,N-ethylmethylacrylamide, N,N-diethylacrylamide, and the like), (meth)acrylamide derivatives having a cyclic group (for example, 1-(1-oxo-2-propenyl)-pyrrolidine, 1-(1-oxo-2-propenyl)-piperidine, 4-(1-oxo-2-propenyl)-morpholine, 1-(1-(oxo-2-methyl-2-propenyl)-pyrrolidine, 1-(1-oxo-2-methyl-2-propenyl)-piperidine, 4-(1-oxo-2-methyl-2-propenyl)-morpholine, and the like), or vinyl ether derivatives (for example, methyl vinyl ether) ; and a photoresponsive material such as a light absorbing polymer having an azobenzene group, a copolymer of a vinyl derivative of triphenylmethane leuco hydroxide and an acrylamide monomer, and N-isopropylacrylamide gel containing spirobenzopyran (for example, refer to JP-A-2-211865 and JP-A-2003-33177), but examples are not limited thereto. It is possible to change their physical properties such as hydrophilicity and hydrophobicity by imparting a predetermined stimulus to these materials, and thereby it is possible to promote detachment of a cell culture adhered onto the materials. Culture dishes coated with temperature-responsive materials are commercially available (for example, UpCell^{(R)} from CellSeed Inc.), and commercially available products thereof can be used in the production method of the present disclosure.

The culture substrate may have various shapes, but it is preferably flat. In addition, an area thereof is not particularly limited, but it is may be, for example, about 1 cm² to about 200 cm², about 2 cm² to about 100 cm², about 3 cm² to about 50 cm², or the like. For example, a circular culture dish having a diameter of 10 cm may be used as the culture substrate. In this case, an area thereof is 56.7 cm².

The culture substrate may be coated (coated or coated) with serum. By using the culture substrate coated with serum, it is possible to form a sheet-shaped cell culture with a higher density. The phrase "coated with serum" means a state in which serum components adhered to a surface of the culture substrate. Such a state is not limited and can be obtained by, for example, treating the culture substrate with serum. A treatment with serum involves bringing serum into contact with the culture substrate and optionally incubating the culture substrate for a predetermined time.

Xenogeneic serum and/or allogeneic serum can be used as serum. The term xenogeneic serum means serum derived from an organism of a species different from that of a recipient in a case where the sheet-shaped cell culture is used for transplantation. For example, in a case where a recipient is human, the xenogeneic serum corresponds to serum derived from bovines or equines, such as fetal bovine serum (FBS, FCS), calf serum (CS), and horse serum (HS). In addition, the term "allogeneic serum" means serum derived from an organism of the same species as that of a recipient. For example, in a case where a recipient is human, the allogeneic serum corresponds to human serum. The allogeneic serum includes autologous serum (also called autologous serum), that is, serum derived from a recipient, and allogeneic serum derived from an allogeneic individual other than the recipient. In the present specification, sera other than autologous serum, that is, xenogeneic serum and allogeneic serum may be collectively referred to as non-autologous serum.

Serum for coating the culture substrate is commercially available, or it can be prepared by a standard method from blood collected from a desired organism. Specific examples of methods include a method in which collected blood is allowed to stand at room temperature for about 20 minutes to about 60 minutes to coagulate, the coagulated blood is centrifuged at about 1000 × g to about 1200 × g, and the supernatant is collected; and the like.

In a case of incubation on the culture substrate, serum may be used as a stock solution or may be diluted and used. Dilution can be performed with any medium such as water, a physiological salt solution, various buffer solutions (for example, PBS, HBSS, and the like), and various liquid media (for example, DMEM, MEM, F12, DMEM/F12, DME, RPMI1640, MCDB (MCDB102, 104, 107, 120, 131, 153, 199, and the like), L15, SkBM, RITC80-7, and the like), but examples of media are not limited thereto. A dilution concentration is not particularly limited as long as serum components can adhere onto the culture substrate, and for example, it is about 0.5% to about 100% (v/v), is preferably about 1% to about 60% (v/v), and is more preferably about 5% to about 40% (v/v).

An incubation time is also not particularly limited as long as serum components can adhere onto the culture substrate, and for example, it is about 1 hour to about 72 hours, is preferably about 2 hours to about 48 hours, is more preferably about 2 hours to about 24 hours, and is even more preferably about 2 hours to about 12 hours. An incubation temperature is also not particularly limited as long as serum components can adhere onto the culture substrate, and for example, it is about 0°C to about 60°C, is preferably about 4°C to about 45°C, and is more preferably a room temperature to about 40°C.

Serum may be discarded after incubation. As a method of discarding serum, a conventional liquid discarding method such as suction with a pipette or decantation can be used. In a preferable aspect of the present disclosure, the culture substrate may be washed with a serum-free washing solution after discarding serum. The serum-free washing solution is not particularly limited as long as it is a liquid medium not containing serum and not adversely affecting serum components adhered to the culture substrate. Washing can be performed using water, a physiological salt solution, various buffer solutions (for example, PBS, HBSS, and the like), various liquid media (for example, DMEM, MEM, F12, DMEM/F12, DME, RPMI1640, MCDB (MCDB102, 104, 107, 120, 131, 153, 199, and the like), L15, SkBM, RITC80-7, and the like), and the like, but examples of serum-free washing solutions are not limited thereto. As a washing method, it is possible to use a conventional culture substrate washing method such as a method in which a serum-free washing solution is added onto a culture substrate, stirred for a predetermined time (for example, about 5 seconds to about 60 seconds), and then discarded, but examples of methods are not limited thereto.

In the present disclosure, the culture substrate may be coated with a growth factor. The term "growth factor" referred to herein means any substance promoting proliferation of cells as compared to a case in which a substance promoting proliferation of cells does not exist, and examples thereof include an epidermal growth factor (EGF), a vascular endothelial growth factor (VEGF), a fibroblast growth factor (FGF), and the like. A method of coating a culture substrate with a growth factor, a method of discarding a growth factor, and a method of washing a growth factor are basically the same methods as the methods for serum except that a dilution concentration at the time of incubation is, for example, about 0.0001 µg/mL to about 1 µg/mL, is preferably about 0.0005 µg/mL to about 0.05 µg/mL, and is more preferably about 0.001 µg/mL to about 0.01 µg/mL.

In the present disclosure, the culture substrate may be coated with a steroid drug. The term "steroid drug" refers to a compound that can exert an adverse effect on a living body such as adrenocortical hypofunction and Cushing syndrome among compounds having a steroid nucleus. Examples of such compounds include, but are not limited to, cortisol, prednisolone, triamcinolone, dexamethasone, betamethasone, and the like. A method of coating a culture substrate with a steroid drug, a method of discarding a steroid drug, and a method of washing a steroid drug are basically the same methods as the methods for serum except that a dilution concentration at the time of incubation is, for example, about 0.1 µg/mL to about 100 µg/mL, is preferably about 0.4 µg/mL to about 40 µg/mL, is more preferably about 1 µg/mL to about 10 µg/mL in the case where a steroid drug is dexamethasone.

The culture substrate may be coated with any one of serum, a growth factor, and a steroid drug, or may be coated with any combination of these, that is, a combination of serum and a growth factor, serum and a steroid drug, serum and a growth factor and a steroid drug, or a growth factor and a steroid drug. In a case of coating with multiple components, these components may be mixed to be applied at the same time, or may be applied in separate steps.

The culture substrate may be seeded with cells immediately after being coated with serum or the like, or may be stored after being coated and then seeded with cells. The coated substrate can be stored for a long period of time by keeping it at, for example, about 4°C or lower, preferably about -20°C or lower, and more preferably about -80°C or lower.

Seeding of cells on the culture substrate can be performed by any known method and condition. Seeding of cells on the culture substrate may be performed by, for example, injecting a cell suspension in which cells are suspended in a culture solution into the culture substrate (culture container) . An instrument suitable for an injection operation of a cell suspension, such as a dropper and a pipette, can be used for injecting the cell suspension.

Seeding can be performed at a density of about 7.1 x 10⁵ cells/cm² to about 3.0 x 10⁶ cells/cm², about 7.3 x 10⁵ cells/cm² to about 2.8 x 10⁶ cells/cm², about 7.5 x 10⁵ cells/cm² to about 2.5 x 10⁶ cells/cm², about 7.8 x 10⁵ cells/cm² to about 2.3 x 10⁶ cells/cm², about 8.0 x 10⁵ cells/cm² to about 2.0 x 10⁶ cells/cm², about 8.5 x 10⁵ cells/cm² to about 1.8 x 10⁶ cells/cm², about 9.0 x 10⁵ cells/cm² to about 1.6 x 10⁶ cells/cm², about 1.0 x 10⁶ cells/cm² to about 1.6 x 10⁶ cells/cm², and the like.

One aspect of the present disclosure relates to a sheet-shaped cell culture for promoting healing of a lumen organ having a damaged part on at least one side of a lumen wall, in which the sheet-shaped cell culture is applied to a corresponding opposite site of the lumen wall of a site in which damage is present.

It is possible to promote healing by applying the sheet-shaped cell culture of the present disclosure to damaged tissue on at least one side (for example, an inner portion) of a lumen wall, from an opposite side (for example, an outer portion) of the lumen wall.

In a case where a lumen wall, for example, one side of a digestive tract wall is damaged, the risk of perforation occurring from the damaged portion increases, and perforation occurs in a case where the damage is thoroughly penetrating damage. In a case where damage is generated on at least one side of a lumen wall, it is possible to promote healing of tissue of lumen organs in which the damage is generated and to regenerate it by applying the sheet-shaped cell culture of the present disclosure to a corresponding opposite side of a site in which the damage is present by, for example, attaching the sheet-shaped cell culture.

As the tissue regeneration mechanism, there are a mechanism by a paracrine effect in which a sheet-shaped cell culture continuously releases cytokines such as VEGF and HGF, and collagen, all of which are responsible for, for example, angiogenesis, cell protection, repair, and the like at a damaged portion; and/or a mechanism by an autocrine effect in which collagen production and the like are promoted by activating precursor cells and stem cells of surrounding tissue to which the sheet-shaped cell culture has been applied.

In the present disclosure, the term "lumen organ" means an organ having a lumen accommodated in a body-cavity, that is, an organ having a tubular or bag-like structure, and examples thereof include organs of the digestive system, the circulatory system, the urinary system, the respiratory system, the female reproductive system, and the like. It is typically an organ of the digestive system. Furthermore, the term "lumen wall" in the present disclosure means an organ wall forming a tube or bag of a lumen organ. A lumen wall has an inner portion facing a lumen and an outer portion forming an outer surface of an organ. In the present disclosure, in a case where the phrase "one side of a lumen wall" is referred to, it means either the inside or the outside of the lumen wall, and the outside with respect to the inside or the inside with respect to the outside is referred to as an "opposite side." Furthermore, a region located at an opposite side of the lumen wall with respect to a certain region that is one side is referred to as a "corresponding opposite side."

Examples of organs of the digestive system include esophagus, stomach, duodenum, pancreas, gallbladder, bile duct, small intestine, large intestine, rectum, and the like. Among them, the duodenum is the most preferable because a lumen wall thereof is thin and therefore the risk of occurrence of perforation is high, and worsening of damage is likely to progress due to a severe environment in which the duodenum is exposed to various digestive fluids.

In one aspect of the present disclosure, the lumen organ is an organ of the digestive system. In organs of the digestive system, a cross-sectional structure of a lumen wall thereof is roughly divided into a three-layer structure of a mucosal layer, a muscular layer, and a serosal layer from a lumen side. The mucosal layer is further classified into a mucosa, a muscularis mucosa, and a submucosal layer, and the serosal layer is further classified into a subserosal layer and a serosa. For example, in a case of a tumor or an ulcer, generally, a lesion is first generated on the inner mucosa, and the lesion invades to the serosal layer side, that is, the outside of the lumen wall, thereby progressing a disease. In a case where the lesion invades to the mucosal layer, the disease can be treated by dissecting and removing the mucosal layer, that is, the mucosa, the muscularis mucosa, and/or the submucosal layer. By such a treatment, a site in which the muscular layer is exposed is generated on the lumen side of the lumen organ. The term "damage" in the present disclosure includes a site in which such a muscular layer is exposed. In addition, in a case where damage reaches the serosa from the mucous for some reason such as a case in which invasion of the lesion reaches the serosa, the damage is thoroughly penetrating damage, that is, penetrating damage. In a case where penetrating damage occurs, it will be a minor leakage such as air leakage if a level of the damage on the serosal side is slight, but it will be a major leakage in which the fluid in the lumen leaks out of the lumen organ if a level of the damage is large.

In the present disclosure, the term "damage" or "damaged part" refers to a state or a part in which at least a part of a lumen wall is impaired. The phrase "having damage on one side" refers to a state in which at least a part of one side (for example, the inner side) of the lumen wall is damaged, where the damage may be thoroughly penetrating damage, that is, it may be penetrating damage of a form in which the lumen wall is damaged by being penetrated, or it may be non-penetrating damage. In a case where the damage is thoroughly penetrating damage, that is, penetrating damage, a damaged part on one side is larger than a damaged part on the other side. For example, in a case of penetrating damage in which a damaged part is at least on an inner side, a damaged part on the inner side is larger than a damaged part on an outer side. In one aspect, damage is non-penetrating damage. In the present disclosure, the term "non-penetrating damage" refers to a state in which damage does not penetrate the lumen wall. That is, in non-penetrating damage occurring on the inner side (lumen side), only the lumen side of the lumen is damaged, and the damage does not reach the outer side (outer wall side). Similarly, in non-penetrating damage occurring on the outer side, only the outer wall side of the lumen is damaged, and the damage does not reach the lumen side. In one aspect, damage is non-penetrating damage in which a mucosal layer of a lumen wall is damaged.

In another aspect, damage is penetrating damage. In a case where the sheet-shaped cell culture of the present disclosure is applied to penetrating damage, it may be applied as it is to a damaged part or a corresponding opposite side of the damaged part, or may be applied after clogging a hole of the penetrating damage by, for example, suturing a damaged site with a thread. Typically, in a case where there is minor leakage among penetrating damages, the sheet-shaped cell culture of the present disclosure is applied as it is, and in a case where there is major leakage among penetrating damages, the sheet-shaped cell culture of the present disclosure is applied after suturing a damaged site.

Damage to the lumen organ may be caused by a wound generated due to an external force such as an incision wound, a lacerated wound, a chop wound, a contused wound, and an avulsed wound, or damage may be caused by, for example, an impaired structure generated due to an ulcer. In the present disclosure, the term "avulsed wound" means a wound generated due to exfoliation of a part of the structure of a lumen organ wall for some reason. In one aspect, the damage of the present disclosure may be a wound such as an incision would and an avulsed wound, and it is preferably an avulsed wound. In another aspect, the damage of the present disclosure may be, for example, damage caused by a procedure on the inner wall of the digestive tract, such as endoscopic submucosal dissection (ESD), endoscopic mucosal resection (EMR), and polypectomy. In a preferable aspect, the damage of the present disclosure is damage caused by a dissection procedure on the inner wall of the digestive tract such as, endoscopic submucosal dissection (ESD) and endoscopic mucosal resection (EMR) . In a more preferable aspect, the damage is damage caused by endoscopic submucosal dissection (ESD).

Accordingly, in a particularly preferable aspect of the present invention, damage is damage cause by ESD of duodenum. As described above, since an intestinal tract is narrow and bent, and a digestive tract wall is thin and is barely within a range of an endoscope, the risk of postoperative perforation occurring at a procedure site as a complication after an ESD procedure is particularly high, because the skill is required to perform the ESD procedure, and a severe environment in which the organ is pancreatic juice and bile are exposed. In many cases, perforation occurring in the stomach and large intestine due to ESD is conservatively healed by tightly closing with a clip. For prevention of postoperative perforation due to ESD of duodenum, methods using existing pharmaceutical products and medical devices have been tried in clinical practice, such as tightly closing with a clip (Maekawa S, et al., Surg Endsc, 2015), and attaching a polyglycolic acid sheet (Neovale sheet ^{(R)}) (Takimoto K, et al., Dig Endosco, 2014), but the current situation is that it has not yet been completely prevented (Fujihara S, et al., World J Gastroenterol, 2016). In contrast, when the sheet-shaped cell culture of the present disclosure is applied to a procedure site of ESD of duodenum from the outer wall side, it is possible to promote regeneration of the procedure site, reduce the risk of postoperative perforation, and treat and/or prevent postoperative perforation. Since inflammation is activated and caused by mixing of pancreatic juice and bile leaking from the lumen wall into the abdominal cavity with digestive fluid, it is possible to prevent inflammation such as peritonitis generated by a surgical treatment such as ESD and postoperative perforation by applying the sheet-shaped cell culture of the present disclosure.

The sheet-shaped cell culture of the present disclosure is applied to at least one of a damaged part or a corresponding opposite part of the damaged part (hereinafter, these may be collectively referred to as an "application site") with respect to damage. In one aspect, the sheet-shaped cell culture of the present disclosure is applied to a corresponding opposite site of a lumen wall of a site in which damage is present. That is, in a case where damage is on an inner portion of a lumen wall, the sheet-shaped cell culture of the present disclosure is applied to a corresponding outer portion of the lumen wall, and in a case where damage is on an outer portion of the lumen wall, the sheet-shaped cell culture of the present disclosure is applied to an corresponding inner portion of the lumen wall.

In another aspect of the present disclosure, the sheet-shaped cell culture of the present disclosure is applied such that the sheet-shaped cell culture entirely covers a damaged part or a corresponding opposite part of the damaged part. The sentence "the sheet-shaped cell culture entirely covers a damaged part or a corresponding opposite part of the damaged part" means that the sheet-shaped cell culture is applied to cover the entire region in which the damaged part is present or the corresponding opposite region thereof. In this case, one sheet-shaped cell culture larger than the region may be applied to cover the entire region, or a plurality of sheet-shaped cell cultures may be applied without gaps to cover the entire region.

Cells constituting the sheet-shaped cell culture of the present disclosure (hereinafter sometimes referred to as "sheet-forming cells") are as described above in detail. The sheet-shaped cell culture of the present disclosure is not required to contain cells of a lumen organ to which it is applied, but it rather may contain cells that are not present in the organ. Accordingly, in one aspect, the sheet-shaped cell culture of the present disclosure contains ectopic cells, that is, cells that are not originally present in an organ to which it is applied. In a preferable aspect, as described above, the sheet-shaped cell culture of the present disclosure is applied to damage of an organ (that is, smooth muscle) of the digestive system, and examples of ectopic cells in this case include cells derived from striated muscle such as skeletal muscle myoblast cells, mesenchymal stem cells, and the like. In a more preferable aspect, the sheet-shaped cell culture of the present disclosure contains skeletal muscle myoblast cells. Skeletal muscle myoblast cells may be derived from striated muscle of a femoral region, a neck region, or an abdominal region.

Skeletal muscle myoblast cells mean myoblast cells present in skeletal muscle (striated muscle) . Skeletal muscle myoblast cells are well known in the present technical field. They can be prepared from skeletal muscle by any known method (for example, a method described in JP-A-2007-89442, and the like), or it is possible to obtain commercially available products thereof (for example, Lonza, Cat# CC-2580) . Skeletal muscle myoblast cells are not limited, and myoblast cells can be identified by, for example, markers such as CD56, α7 integrin, myosin heavy chain IIa, myosin heavy chain IIb, myosin heavy chain IId (IIx), MyoD, Myf5, Myf6, myogenin, desmin, and PAX3. In a specific aspect, skeletal muscle myoblast cells are CD56-positive. In a more specific aspect, skeletal muscle myoblast cells are CD56-positive and desmin-positive. Skeletal muscle myoblast cells are any organisms that have skeletal muscle, and they may be derived from humans, non-human primates, rodents (mice, rats, hamsters, guinea pigs, and the like), mammals such as rabbits, dogs, cats, pigs, horses, cows, goats, and sheep, but examples are not limited thereto. In one aspect, skeletal muscle myoblast cells are skeletal muscle myoblast cells of mammals. In a specific aspect, skeletal muscle myoblast cells are skeletal muscle myoblast cells of human.

In a case where skeletal muscle myoblast cells are prepared from striated muscle tissue, the prepared cell population contains fibroblast cells . In a case where the cell population containing skeletal muscle myoblast cells which is prepared from striated muscle tissue is used in the production of the sheet-shaped cell culture of the present disclosure, the cell population contains a certain amount of fibroblast cells. Fibroblast cells are well known in the present technical field, and they can be identified by, for example, markers such as TE-7 (for example, refer to Rosendaal et al., J Cell Sci. 1994; 107 (Pt 1): 29-37, Goodpaster et al., J Histochem Cytochem. 2008; 56(4): 347-58, and the like).

In one aspect, cells forming the sheet-shaped cell culture of the present disclosure include skeletal muscle myoblast cells prepared from striated muscle tissue. Accordingly, a cell population to be used in the production of the sheet-shaped cell culture of the present disclosure may contain skeletal muscle myoblast cells and fibroblast cells. In one aspect, a CD56-positive rate of the cell population used in the production of the sheet-shaped cell culture of the present disclosure is 50% or more, 60% or more, 65% or more, 70% or more, 75% or more, 80% or more, 85% or more, and 90% or more, and it is preferably 60% or more.

The cell population used in the production of the sheet-shaped cell culture of the present disclosure may contain fibroblast cells, but in a case where a content of fibroblast cells is too high, it is not preferable because then a content of skeletal muscle myoblast cells decreases. Accordingly, in one aspect, a TE7-positive rate of the cell population used in the production of the sheet-shaped cell culture of the present disclosure is 50% or less, 40% or less, 35% or less, 30% or less, 25% or less, 20% or less, 15% or less, and 10% or less, and it is preferably 40% or less.

The cell population used in the production of the sheet-shaped cell culture of the present disclosure may contain cells other than skeletal muscle myoblast cells and fibroblast cells, but it is preferable that the number of such cells is as small as possible. Accordingly, a total value of the CD56-positive rate and the TE7-positive rate is preferably high, and for example, it is 80% or more, 85% or more, 90% or more, 95% or more, 96% or more, 97% or more, 98% or more, 99% or more, or the like, and it is preferably 90% or more.

A thickness of the sheet-shaped cell culture of the present disclosure is not particularly limited. In a case where a single-layered sheet is used as the sheet-shaped cell culture, a thickness thereof is generally a thickness of one cell or more, and the thickness varies depending on the type of sheet-forming cell. In one aspect, a thickness of the sheet-shaped cell culture is 30 µm or more, and in a preferable aspect, a thickness of the sheet-shaped cell culture is 50 µm or more. A thickness range of the sheet-shaped cell culture of the present disclosure is, for example, 30 µm to 200 µm, is preferably 50 µm to 150 µm, and is more preferably 60 µm to 100 µm or the like. In a case where a laminate sheet is used as the sheet-shaped cell culture, a thickness thereof is set such that it does not exceed a thickness of the single-layered sheet × the number of laminated layers. Accordingly, in a case of using a sheet in which five single-layered sheets are laminated is used for example as one aspect, a thickness thereof is 150 µm or more, is preferably 250 µm or more, and a thickness range is, for example, 150 µm to 1000 µm, is preferably 250 µm to 750 µm, and is more preferably 300 µm to 500 µm or the like.

Accordingly, a thickness of the sheet-shaped cell culture of the present disclosure is, for example, 30 µm to 1000 µm, and is preferably 50 µm to 750 µm, 50 µm to 500 µm, 60 µm to 500 µm, or the like.

In some aspects, the sheet-shaped cell cultures of the present disclosure may be very fragile and difficult to handle. Accordingly, the sheet-shaped cell culture of the present disclosure may further have a reinforcement layer for the purpose of ease of handling and reducing the risk of breakage. The reinforcement layer may be any layer as long as it can reinforce the structure without impairing the function of the sheet-shaped cell culture of the present disclosure. It may be a reinforcement layer containing, for example, a gel and/or polymer, but since it is to be transplanted into a living body, it is preferably a biocompatible reinforcement layer containing, for example, a biocompatible gel or polymer.

The gel, preferably a biocompatible gel, that can be used for the reinforcement layer of the present disclosure may be any gel as long as it does not adversely affect a living body when it is introduced into the living body. Examples thereof include, but are not limited to, a fibrin gel, a fibrinogen gel, a gelatin gel, a collagen gel, and the like.

The polymer, preferably a biocompatible polymer, that can be used in the reinforcement layer of the present disclosure is not limited and may be any polymer that does not adversely affect a living body when it is introduced into the living body. Examples thereof include, but are not limited to, polylactic acid, polyglycolic acid, polydioxano, polyglycapro, collagen, and the like.

As a method of forming a reinforcement layer containing a biocompatible gel, methods known in the present technical field can be used. Examples of such methods include, but are not limited to, a method of spraying a biocompatible gel or polymer, or a component serving as a material thereof on a sheet-shaped cell culture; a method of laminating a sol-like biocompatible substance on a sheet-shaped cell culture and gelling the same; a method of immersing a biocompatible gel or polymer in a liquid gel and then solidifying a gel; a method disclosed in JP-A-2016-52271; and the like.

Since the reinforcement layer is for ease of handling of the sheet-shaped cell culture of the present disclosure and reducing the risk of breakage, the reinforcement layer preferably has a certain strength or more, and furthermore, it preferably has elasticity. A known evaluation unit for strength of a structure containing a gel or polymer is, for example, jelly strength, and the like, and a known evaluation unit for strength of a sheet-shaped structure is, for example, a tensile breaking load, and the like. A method of measuring jelly strength is described in, for example, JIS K 6503, and the like. A tensile breaking load means the maximum load until both ends of a sheet-shaped cell culture or the like are pulled in a horizontal direction to break, and a measuring method thereof is described in, for example, JP-A-2014-179151, and the like.

A tensile breaking load of the reinforcement layer of the sheet-shaped cell culture of the present disclosure may be about 0.010N or more, about 0.015N or more, about 0.020N or more, about 0.025N or more, about 0.030N or more, about 0.035N or more, about 0.040N or more, about 0.045N or more, and the like; or it may range from about 0.010N to about 0.200N, about 0.015N to about 0.100N, about 0.020N to about 0.50N, and the like, but examples of tensile breaking loads and ranges thereof are not limited thereto. Compared to a sheet-shaped cell culture not having the reinforcement layer, a strength of the sheet-shaped cell culture having the reinforcement layer may be about 1.5 times or more, about 2 times or more, about 3 times or more, about 4 times or more, about 5 times or more, about 6 times or more, about 7 times or more, about 8 times or more, about 9 times or more, and about 10 times or more; or it may range from about 1.5 times to about 20 times, about 2 times to about 15 times, about 2.5 times to about 10 times, and the like.

In a case where the sheet-shaped cell culture having the reinforcement layer is applied, it is preferably applied such that the reinforcement layer does not come into direct contact with the application site. That is, the sheet-shaped cell culture is preferably applied such that it is located between the application site and the reinforcement layer.

Application of the sheet-shaped cell culture to the application site can be performed using any device and/or method known in the present technical field.

In one aspect, in a case where the sheet-shaped cell culture of the present disclosure is applied to lumen tissue, it may be applied in combination with other compositions and/or grafts which promote healing. Examples of other compositions and/or grafts which promote healing include, but are not limited to, grafts containing pedunculated blood vessels such as pedunculated omentum grafts, polyglycolic acid sheets, fibrin gels, Ad-spray^{(R)}, and the like. In a preferable aspect, the sheet-shaped cell culture of the present disclosure is applied together with a graft containing pedunculated blood vessels. Typical examples of grafts containing pedunculated blood vessel include a pedunculated omentum graft and the like . Such other compositions and/or grafts which promote healing may be different compositions or grafts than the sheet-shaped cell culture of the present disclosure, and they may be substances incorporated in, for example, the sheet-shaped cell culture, the reinforcement layer, or the like.

In a case where the sheet-shaped cell culture of the present disclosure is applied together with different other compositions and/or grafts which promote healing, such compositions and/or grafts may be applied before application of the sheet-shaped cell culture or may be applied after the application. In a case where the other compositions and/or grafts are applied before the application of the sheet-shaped cell culture, they are applied such that they are located between the application site and the sheet-shaped cell culture. That is, first, the other compositions and/or grafts are applied to the application site, and thereafter, the sheet-shaped cell culture (optionally containing the reinforcement layer) is applied thereto. In a case where the other compositions and/or grafts are applied after the application of the sheet-shaped cell culture, they are applied on the sheet-shaped cell culture (optionally containing the reinforcement layer) which is on the application site. That is, first, the sheet-shaped cell culture is applied to the application site, and thereafter, the other compositions and/or grafts are applied thereto.

Another aspect of the present disclosure relates to a method for producing the sheet-shaped cell culture of the present disclosure (hereinafter, also referred to as a "production method of the present disclosure").

The production method of the present disclosure includes:
step (i) of seeding a cell population containing sheet-forming cells to a culture substrate;
step (ii) of sheet-forming of the cell population seeded in step (i) in a sheet culture medium to form a sheet-shaped cell culture; and
step (iii) of detaching the sheet-shaped cell culture formed in step (ii) from the culture substrate.

In (i), the cell population containing the sheet-forming cells is seeded on the culture substrate. The sheet-forming cells are not particularly limited as long as they are the above-described cells as cells that constitute the sheet-shaped cell culture. The cell population contains at least one type of sheet-forming cell, but it may contain two or more types of sheet-forming cell or may contain cells other than the sheet-forming cells. In one aspect of the present disclosure, at least one type of sheet-forming cell contained in the cell population is an ectopic cell that is not present in an application organ, and it is preferably a skeletal muscle myoblast cell. In such an aspect, the cell population can further contain fibroblast cells. That is, a sheet-shaped cell culture containing skeletal muscle myoblast cells and fibroblast cells as sheet-forming cells can be exemplified. In another aspect of the present disclosure, at least one type of sheet-forming cell contained in the cell population is a mesenchymal stem cell. In such an aspect, the cell population can further contain vascular endothelial cells and cardiomyocytes.

A density of cells to be seeded is not particularly limited as long as it is a density that enables formation of the sheet-shaped cell culture, but in a preferable aspect, the cell population is seeded at a density at which cells reach confluence or a higher density. In the present disclosure, the phrase "density at which cells reach confluence" refers to a density at which, when cells are seeded, the seeded cells are assumed to cover the entire adhesive surface of a culture container without any gap. For example, the phrase refers to a density at which cells are assumed to come into contact with each other, a density at which contact inhibition occurs, or a density at which cell proliferation is substantially stopped due to contact inhibition, when cells are seeded.

The "density at which cells reach confluence or a higher density" may vary depending on the type of sheet-forming cell to be seeded. For example, in a case of skeletal muscle myoblast cells, a density may be 3.0 × 10⁵ cells/cm² or more, 3.5 × 10⁵ cells/cm² or more, 1.0 × 10⁶ cells/cm² or more, and the like.

Non-limiting examples of seeding densities of the cell population include a density of about 7.1 x 10⁵ cells/cm² to about 3.0 x 10⁶ cells/cm², about 7.3 x 10⁵ cells/cm² to about 2.8 x 10⁶ cells/cm², about 7.5 x 10⁵ cells/cm² to about 2.5 x 10⁶ cells/cm², about 7.5 x 10⁵ cells/cm² to about 3.0 x 10⁶ cells/cm², about 7.8 x 10⁵ cells/cm² to about 2.3 x 10⁶ cells/cm², about 8.0 x 10⁵ cells/cm² to about 2.0 x 10⁶ cells/cm², about 8.5 x 10⁵ cells/cm² to about 1.8 x 10⁶ cells/cm², about 9.0 x 10⁵ cells/cm² to about 1.6 x 10⁶ cells/cm², about 1.0 x 10⁶ cells/cm² to about 1.6 x 10⁶ cells/cm², and the like. These densities are densities of all cells contained in the cell population, unless otherwise specified.

In still another aspect, seeding can be performed in a cell culture solution substantially free of growth factors at a density at which at least one type of sheet-forming cell that may be contained in the cell population does not substantially proliferate. In such an aspect, other cells that may be contained in the cell population may be at a proliferative density while undergoing proliferation suppression.

The culture substrate used in the method of the present disclosure is as described above. In a preferable aspect, the culture substrate may be coated with serum. In another preferable aspect, the culture substrate may be coated with a temperature-responsive material. In still another preferable aspect, the culture substrate may be coated with a temperature-responsive material and serum.

In (ii), the seeded cell population is incubated in the sheet culture medium for sheet-forming, and thereby is formed as the sheet-shaped cell culture.

Sheet-forming of the seeded cells can be performed by any known method and condition. Non-limiting examples of such methods are disclosed in, for example, PTL 1, WO2014/185517, and the like. It is considered that sheet-forming of cells is achieved by cells adhering to each other via an adhesion molecule or cells adhering to each other via an intercellular adhesion mechanism such as an extracellular matrix. Accordingly, sheet-forming of the seeded cells can be achieved by, for example, culturing cells under conditions in which cell-cell adhesion is formed. Such conditions may be any conditions as long as cell-cell adhesion is formed thereunder, but normally cell-cell adhesion can be formed under the same conditions as general cell culture conditions. Examples of such conditions include culturing at about 37°C and 5% CO₂. In addition, culturing can be performed under normal pressure (under atmospheric pressure or under no pressure). Culturing can be performed in a container of any size and shape. A size and a shape of the sheet-shaped cell culture can be optionally adjusted by adjusting a size and a shape of a cell attachment surface of a culture container, or by installing a mold of a desired size and shape on a cell attachment surface of a culture container and culturing cells therein. In the present specification, culturing for sheet-forming of the seeded cells may be referred to as "sheet-forming incubation." The sheet-forming incubation reduces a thickness of a sheet-shaped cell culture on a culture substrate (in a culture container) . That is, a thickness of a cell layer on a culture substrate is reduced after seeding, after precipitating cells, and after subsequent sheet-forming, but the sheet-shaped cell culture shrinks after being detached from the culture substrate, and a thickness thereof increases again. A reduction in thickness due to sheet-forming is about 90% to about 70% in a case where a thickness of a cell layer immediately after seeding is defined as 100%.

It is desirable that incubation for sheet-forming be terminated before the sheet-shaped cell culture is spontaneously detached. A time from the start of incubation to the start of spontaneous detachment may vary depending on the type of cell contained in the seeded cell population (particularly the type of sheet-forming cell) and a state of cells, but spontaneous detachment occurs in about 6 to 12 hours in many cases when a cell population containing skeletal muscle myoblast cells as sheet-forming cells is seeded. Accordingly, in one aspect of the present disclosure, the upper limit of an incubation time for sheet-forming may be 12 hours, 11.5 hours, 11 hours, 10 hours, 9 hours, 8 hours, 7 hours, 6 hours, 5 hours, or 4 hours. Therefore, in the production method of the present disclosure, an incubation time for sheet-forming may be 2 to 12 hours, 2 to 11.5 hours, 2 to 11 hours, 2 to 10 hours, 2 to 9 hours, 2 to 8 hours, 2 to 7 hours, 2 to 6 hours, 2 to 5 hours, or 2 to 4 hours, and it is preferably 2 to 4 hours or 2 to 6 hours.

A cell culture solution used for sheet-forming incubation (sometimes referred to as a "sheet culture medium" or simply referred to as a "culture solution" or a "medium") is not particularly limited as long as it can maintain cell survival, but typically, it is possible to use a cell culture solution having an amino acid, a vitamin, and an electrolyte as main components. In one aspect of the present disclosure, the culture solution is based on a basal medium for cell culture. Examples of basal media include, but are not limited to, DMEM, MEM, F12, DMEM/F12, DME, RPMI1640, MCDB (MCDB102, 104, 107, 120, 131, 153, 199, and the like), L15, SkBM, RITC80-7, and the like. Many of these basal media are commercially available and their compositions are known.

The basal medium may be used as it is as a standard composition (for example, as it is on the market), or its composition may be appropriately changed depending on the cell type and cell conditions. Accordingly, the basal medium used in the present disclosure is not limited to a basal medium having a known composition, and includes a basal medium in which one or two or more components have been added, removed, increased, or decreased.

Examples of amino acids contained in the basal medium include, but are not limited to, L-arginine, L-cystine, L-glutamine, glycine, L-histidine, L-isoleucine, L-leucine, L-lysine, L-methionine, L-phenylalanine, L-serine, L-threonine, L-tryptophan, L-tyrosine, L-valine, and the like. Examples of vitamins include, but are not limited to, D-calcium pantothenate, choline chloride, folic acid, i-inositol, niacinamide, riboflavin, thiamine, pyridoxine, biotin, lipoic acid, vitamin B12, adenine, thymidine, and the like. Examples of electrolytes include, but are not limited to, CaCl₂, KCl, MgSO₄, NaCl, NaH₂PO₄, NaHCO₃, Fe(NO₃)₃, FeSO₄, CuSO₄, MnSO₄, Na₂SiO₃, (NH₄)₅Mo₇O₂₄, NaVO₃, NiCl₂, ZnSO₄, and the like. In addition to these components, the basal medium may contain saccharides such as D-glucose, sodium pyruvate, pH indicators such as phenol red, putrescine, and the like.

In one aspect of the present disclosure, a concentration of an amino acid contained in the basal medium is as follows: L-arginine: about 63.2 mg/L to about 84 mg/L; L-cystine: about 35 mg/L to about 63 mg/L; L-glutamine: about 4.4 mg/L to about 584 mg/L; glycine: about 2.3 mg/L to about 30 mg/L; L-histidine: about 42 mg/L; L-isoleucine: about 66 mg/L to about 105 mg/L; L-leucine: about 105 mg/L to about 131 mg/L; L-lysine: about 146 mg/L to about 182 mg/L; L-methionine: about 15 mg/L to about 30 mg/L; L-phenylalanine: about 33 mg/L to about 66 mg/L; L-serine: about 32 mg/L to about 42 mg/L; L-threonine: about 12 mg/L to about 95 mg/L; L-tryptophan: about 4.1 mg/L to about 16 mg/L; L-tyrosine: about 18.1 mg/L to about 104 mg/L; and L-valine: about 94 mg/L to about 117 mg/L.

In addition, in one embodiment of the present disclosure, a concentration of a vitamin preparation contained in the basal medium is as follows: D-calcium pantothenate: about 4 mg/L to about 12 mg/L; choline chloride: about 4 mg/L to about 14 mg/L; folic acid: about 0.6 mg/L to about 4 mg/L; i-inositol: about 7.2 mg/L; niacinamide: about 4 mg/L to about 6.1 mg/L; riboflavin: about 0.0038 mg/L to about 0.4 mg/L; thiamine: about 3.4 mg/L to about 4 mg/L; and pyridoxine: about 2.1 mg/L to about 4 mg/L.

In addition to the above examples, the cell culture solution may contain one or two or more additives such as serum, growth factors, steroid drug components, and selenium components. However, in a case where these components are not autologous, because clinically, they can be undeniable impurities derived from production steps that can be a side effect factor such as anaphylactic shock to a recipient, it may be desirable to exclude such non-autologous components for clinical applications. Accordingly, in a preferable aspect of the present disclosure, the cell culture solution does not contain an effective amount of at least one of these non-autologous additives. Furthermore, in a more preferable aspect of the present disclosure, the cell culture solution is substantially free of at least one of these non-autologous additives. Furthermore, in a particularly preferable aspect of the present disclosure, the cell culture solution is substantially free of non-autologous additives . In one aspect, the cell culture solution may contain only a basal medium.

In one aspect of the present disclosure, the cell culture solution is substantially free of serum. The cell culture solution substantially free of serum may be referred to as a "serum-free medium" in the present specification. The phrase "substantially free of serum" means that an amount of serum in the culture solution is about an amount not exerting an adverse effect in a case where the sheet-shaped cell culture is applied to a living body (for example, an amount of serum albumin in the sheet-shaped cell culture is less than about 50 ng), and preferably means that these substances are not actively added to the culture solution. In the present disclosure, the cell culture solution is preferably substantially free of xenogeneic serum, and is more preferably substantially free of non-autologous serum in order to avoid side effects during transplantation.

In one aspect of the disclosure, the cell culture solution contains serum. The serum may be allogeneic serum or xenogeneic serum. In a particular aspect, the cell culture solution contains autologous serum. When culturing cells on a culture substrate coated with serum, the serum contained in the cell culture solution (serum used for culturing cells) may be the same as or different from the serum used for coating the culture substrate. In one aspect, the serum contained in the cell culture solution is the same as the serum used to coat the culture substrate, and in a specific aspect, the serum is autologous serum. The serum may be for use in the production method of the present disclosure. For example, the serum may be for use in culturing cells or for coating a culture substrate.

In one aspect of the present disclosure, the cell culture solution does not contain an effective amount of growth factors. The phrase "effective amount of growth factors" means an amount of growth factors that significantly promotes the proliferation of cells as compared with the case where no growth factor is used, or means, for convenience, an amount usually added for the purpose of proliferation of cells in the present technical field. The significance of cell proliferation promotion can be appropriately evaluated by, for example, any statistical method known in the present technical field, such as t-test, and the usual addition amount can be known from various published documents in the present technical field. Specifically, an effective amount of EGF in cell culture is, for example, about 0.005 µg/mL or more.

Accordingly, the phrase "does not contain an effective amount of growth factors" means that the concentration of the growth factor in the culture solution in the present disclosure is less than such an effective amount. For example, a concentration of EGF in the culture solution in cell culture is preferably less than about 0.005 µg/mL, and more preferably less than about 0.001 µg/mL. In a preferable aspect of the present disclosure, a concentration of the growth factor in the culture solution is lower than a normal concentration in a living body. In such an aspect, for example, a concentration of EGF in the culture solution in cell culture is preferably less than about 5.5 ng/mL, more preferably less than about 1.3 ng/mL, and even more preferably less than about 0.5 ng/mL. In a more preferable aspect, the culture solution in the present disclosure is substantially free of growth factors. The phrase "substantially free of" means that a content of the growth factor in the culture solution is such that it does not exert an adverse effect in a case where the sheet-shaped cell culture is applied to a living body, and preferably means that the growth factor is not actively contained in the culture solution. Accordingly, in this aspect, the culture solution does not contain a growth factor at a concentration higher than that contained in other components therein, such as serum.

In one aspect of the present disclosure, the cell culture solution is substantially free of steroid drug components. The term "steroid drug component" refers to a compound that can exert an adverse effect on a living body such as adrenocortical hypofunction and Cushing syndrome among compounds having a steroid nucleus. Examples of such compounds include, but are not limited to, cortisol, prednisolone, triamcinolone, dexamethasone, betamethasone, and the like. Accordingly, the phrase "substantially free of steroid drug component" means that a content of these compounds in the culture solution is such that it does not adversely affect the application of the sheet-shaped cell culture to a living body, and preferably means that these compounds are not actively added to the culture solution, that is, the culture solution does not contain other components therein, for example, steroid drug components at concentrations higher than those contained in serum and the like.

In one aspect of the present disclosure, the cell culture solution is substantially free of selenium components. The term "selenium component" includes a selenium molecule and a selenium-containing compound, particularly a selenium-containing compound capable of liberating the selenium molecule in vivo, such as selenious acid. Accordingly, the phrase "substantially free of selenium components" means that a content of these substances in the culture solution is such that it does not exert an adverse effect in a case where the sheet-shaped cell culture is applied to a living body, and preferably means that these substances are not actively added to the culture solution, that is, the culture solution does not contain other components therein, for example, selenium components at concentrations higher than those contained in serum and the like. Specifically, for example, in the case of human, a selenium concentration in the culture solution is lower than a value obtained by multiplying a normal value in human serum (for example, 10.6 µg/dL to 17.4 µg/dL) by a ratio of human serum contained in the medium (that is, when a content of human serum is about 10%, a selenium concentration is, for example, about 1.0 µg/dL to less than about 1.7 µg/dL).

In the above preferable aspect of the present disclosure, a step of removing impurities by washing or the like is not required, the impurities being derived from the production process such as growth factors, steroid drug components, and xenogeneic serum components, which are conventionally required in a case of producing a cell culture to be applied to a living body. Accordingly, one aspect of the method of the present disclosure does not include the step of removing impurities derived from a production process.

The term "impurities derived from a production process" typically includes substances listed below which are derived from each production process. That is, there are substances derived from cell substrates (for example, host cell-derived proteins, host cell-derived DNA), substances derived from cell culture solution (for example, inducer, antibiotics, media components), substances derived from the extraction, separation, processing, and purification steps of a target substance, which are steps performed after cell culture, or the like (refer to, for example, Pharmaceutical Examination No. 571).

In (iii), the formed sheet-shaped cell culture is detached from the culture substrate.

Detachment of the sheet-shaped cell culture from the culture substrate is not particularly limited as long as the sheet-shaped cell culture can be released (detached) from the culture substrate that is the scaffold at least partially while maintaining the sheet structure. For example, it can be carried out by an enzymatic treatment with a proteolytic enzyme (for example, trypsin and the like) and/or a mechanical treatment such as pipetting. In addition, in a case where cells are cultured to form a cell culture by culturing cells on a culture substrate having a surface coated with a material having physical properties that change in response to a stimulus, for example, temperature or light, the sheet-shaped cell culture can also be released non-enzymatically by applying a predetermined stimulus.

For example, in a case where cells are cultured in a temperature-responsive culture dish to form a cell culture, the sheet-shaped cell culture can be released non-enzymatically by performing a temperature treatment in which a temperature is set to be equal to or lower than the lower critical solution temperature (LCST) or equal to or higher than the upper critical solution temperature (UCST) with respect to water of the temperature-responsive material. Such a temperature treatment is not limited, and can be achieved by, for example, by transferring a culture environment of a culture substrate to which the formed sheet-shaped cell culture adhered from a culture environment at a temperature higher than LCST (for example, in an incubator at a temperature of about 37°C) to an environment at LCST or lower (for example, a room temperature environment outside the incubator and the like) . The transition to the environment of LCST or lower is not limited, and it can be achieved by, for example, substituting a culture solution having a temperature higher than LCST in which the formed sheet-shaped cell culture is present by a medium at a temperature of LCST or lower (for example, buffer solutions (PBS, HBSS, and the like), liquids such as culture solutions), and the like). Accordingly, the medium such as the buffer solution can be used for non-enzymatically releasing the sheet-shaped cell culture from the culture substrate in the production method of the present disclosure.

The sheet-shaped cell culture detached in step (iii) shrinks as compared to before the detachment, and thereby an area thereof becomes smaller. The sheet-shaped cell culture produced by the production method of the present disclosure is characterized by being less likely to shrink after detachment and having a larger area. In one aspect of the present disclosure, the sheet-shaped cell culture after detachment has an area of about 20% or more, for example, about 20%, about 25%, about 30%, about 31%, about 32%, about 33%, about 34%, about 35%, about 36%, about 37%, about 38%, about 39%, about 40%, about 50%, or about 60%, and it preferably has an area of about 35% or more, for example, about 35%, about 36%, about 37%, about 38%, about 39%, about 40%, about 50%, or about 60%, with respect to an area of the sheet-shaped cell culture before detachment (that is, an area of the culture substrate).

The production method of the present disclosure may include a step of freezing cells (cell population) and a step of thawing frozen cells before (i). Freezing of cells can be performed by any known method. Examples of such methods include, but are not limited to, subjecting cells in a container to a freezing means such as a freezer, a deep freezer, and a low temperature medium (for example, liquid nitrogen and the like), and the like. A temperature of the freezing means is not particularly limited as long as it can freeze a part, preferably the whole, of the cell population in the container, but it is typically about 0°C or lower, preferably about -20°C or lower, more preferably about -40°C or lower, and even more preferably about -80°C or lower. In addition, a cooling rate in the freezing operation is not particularly limited as long as it does not significantly impair a survival rate and functions of cells after freeze-thawing, but a cooling rate is typically about 1 hour to about 5 hours, preferably about 2 hours to about 4 hours, and particularly about 3 hours in a case where cooling starts at 4°C and a temperature reaches about -80°C. Specifically, for example, cooling can be performed at a rate of about 0.46°C/min. Such a cooling rate can be achieved by directly supplying the container containing the cells to the freezing means set to a desired temperature or by accommodating the container in a freezing treatment container. The freezing treatment container may have a function of controlling a rate of decrease of a temperature in the container to a predetermined rate. As the freezing treatment container, any known container can be used, and it is possible to use, for example, BICELL^{(R)} (Japan Freezer), program freezer, and the like.

The freezing operation may be performed while cells are immersed in a culture solution or a physiological buffer solution, but it may be performed after adding a cryoprotective agent for protecting cells from the freezing/thawing operation to the culture solution, or after subjecting the culture solution to a treatment such as replacement with a cryopreservation solution containing the cryoprotective agent. Accordingly, the production method of the present disclosure including the freezing step may further include a step of adding a cryoprotective agent to the culture solution, or a step of replacing the culture solution with a cryopreservation solution. In a case of replacing the culture solution with a cryopreservation solution, when the solution in which the cells are immersed at the time of freezing contains a cryoprotective agent at an effective concentration, the cryopreservation solution may be added after removing the culture solution substantially completely, or the cryopreservation solution may be added while leaving a part of the culture solution. The "effective concentration" referred to herein means that a concentration at which the cryoprotective agent exerts a cryoprotective effect, for example, an effect of suppressing the decrease in cell survival rate, vitality, function and the like after freeze-thawing without showing toxicity, as compared to a case in which a cryoprotective agent is not used. Such a concentration is known to those skilled in the art or can be appropriately determined by routine experiments and the like.

The cryoprotective agent is not particularly limited as long as it has a cryoprotective effect on cells, and examples thereof include dimethyl sulfoxide (DMSO), glycerol, ethylene glycol, propylene glycol, sericin, propanediol, dextran, polyvinylpyrrolidone, polyvinyl alcohol, hydroxyethyl starch, chondroitin sulfate, polyethylene glycol, formamide, acetamide, adonitol, perseitol, raffinose, lactose, trehalose, sucrose, mannitol, and the like. The cryoprotective agent may be used alone or in combination of two or more kinds thereof.

A concentration of the cryoprotective agent added to the culture solution or a concentration of the cryoprotective agent in the cryopreservation solution is not particularly limited as long as it is an effective concentration as defined above, and it is typically, for example, about 2% to about 20% (v/v) with respect to the whole culture solution or cryopreservation solution. However, it is also possible to employ alternative use concentrations known or experimentally determined for each cryoprotective agent although a range thereof is out of this concentration range, and such concentrations are also within the scope of the present disclosure.

The step of thawing the frozen cells can be performed by any known cell thawing technique, and can be typically achieved by, for example, subjecting frozen cells to thawing means, for example, a solid, liquid or gaseous medium having a temperature higher than the freezing temperature (for example, water), a water bath, an incubator, an incubator, or the like, or by immersing the frozen cells in a medium (for example, a culture solution) having a temperature higher than a freezing temperature, but methods are not limited thereto. A temperature of the thawing means or the immersion medium is not particularly limited as long as it can thaw the cells within a desired time, but it is typically about 4°C to about 50°C, preferably about 30°C to about 40°C, and more preferably about 36°C to about 38°C. A thawing time is not particularly limited as long as it does not significantly impair a survival rate or function of the cells after thawing, but it is typically about 2 minutes or less, and particularly, when it is about 20 seconds or less, it is possible to significantly suppress a decrease in survival rate. A thawing time can be adjusted, for example, by changing a temperature of the thawing means or the immersion medium, a volume or composition of the culture solution or the cryopreservation solution at the time of freezing. Frozen cells include cells frozen by any technique, and non-limiting examples thereof include cells frozen by the above-mentioned step of freezing cells, and the like. In one aspect, the frozen cells are cells frozen in the presence of the cryoprotective agent. In one aspect, the frozen cells are for use in the production method of the present disclosure.

The production method of the present disclosure includes a step of washing the cells after the above-described step of thawing the frozen cells and before the step of forming a sheet-shaped cell culture, preferably the step of seeding cells in a culture substrate. Washing of cells can be carried out by any known method, and can be achieved typically by, for example, suspending cells in a culture solution (for example, medium and the like) or a physiological buffer solution (for example, PBS, HBSS, and the like), which contains or does not contain a washing solution (for example, serum or serum components (serum albumin, and the like)), and performing centrifugal separation, discarding the supernatant, and recovering the precipitated cells, but methods are not limited thereto. In the step of washing cells, a cycle of such suspension, centrifugal separation, and recovery may be performed once or plural times (for example, 2, 3, 4, 5 times, or the like) . In one aspect of the present disclosure, washing cells is performed immediately after thawing the frozen cells.

The production method of the present disclosure may further include a step of proliferating cells before the above-mentioned step of freezing the cells. The step of proliferating cells may be performed by any known method, and those skilled in the art are familiar with culture conditions suitable for proliferating various cells.

In one aspect, the production method of the present disclosure does not include a step of introducing a gene into a cell. In another aspect, the production method of the present disclosure includes the step of introducing a gene into a cell. A gene to be introduced is not particularly limited as long as it is useful for treating a target disease, and it may be, for example, a cytokine such as HGF or VEGF. Genes can be introduced using any known method such as a calcium phosphate method, a lipofection method, a ultrasound introduction method, an electroporation method, a particle gun method, a method using viral vectors such as an adenovirus vector and a retrovirus vector, a microinjection method, and the like. Introduction of a gene into a cell can be performed, for example, before the step of freezing the cells, but there is no limitation.

In one aspect, all the steps of the production method of the present disclosure are performed in vitro. In another aspect, the production method of the present disclosure includes a step performed in vivo, for example, a step of collecting a cell, or a tissue from which the cell is obtained (for example, striated muscle tissue, particularly skeletal muscle tissue) from a target, but there is no limitation. In one aspect, all the steps of the production method of the present disclosure are performed under aseptic conditions. In one aspect, the production method of the present disclosure is performed such that the sheet-shaped cell culture obtained last is substantially sterile. In one aspect, the production method of the present disclosure is performed such that the sheet-shaped cell culture obtained last is sterile.

The production method of the present disclosure may optionally include a step of forming a reinforcement layer that is a biocompatible gel on a detached sheet-shaped cell culture after step (iii). The biocompatible gel that can be used to form the reinforcement layer is as described in detail above.

As a method of forming a reinforcement layer containing a biocompatible gel, methods known in the present technical field can be used. Examples of such methods include, but are not limited to, a method of spraying a biocompatible gel on a sheet-shaped cell culture; a method of laminating a sol-like biocompatible substance on a sheet-shaped cell culture and gelling the same; a method of immersing a biocompatible gel or polymer in a liquid gel and then solidifying a gel; a method disclosed in JP-A-2016-52271; and the like.

Another aspect of the present disclosure relates to a composition (for example, a pharmaceutical composition and the like), a graft, a medical product, and the like (hereinafter, collectively referred to as a "composition and the like"), which contain the sheet-shaped cell culture of the present disclosure.

The composition and the like of the present disclosure include various additional components in addition to the sheet-shaped cell culture of the present disclosure, for example, a pharmaceutically acceptable carrier; components that enhance viability, engraftment and/or function of the sheet-shaped cell culture; other active components and/or grafts useful for regeneration of application organs; and the like. As such additional components, any known component can be used, and those skilled in the art are familiar with these additional components. In addition, the composition and the like of the present disclosure can be used together with the components that enhance viability, engraftment and/or function of the sheet-shaped cell culture; the other active components and/or grafts useful for promoting regeneration and healing of application organs; and the like.

Another aspect of the present disclosure relates to the above-described method of promoting healing of tissue of a lumen organ having a damaged part on at least one side of a lumen wall in a target, the method including applying an effective amount of the sheet-shaped cell culture of the present disclosure or a composition or the like to a corresponding opposite site of the lumen wall of a site in which damage is present (hereinafter, sometimes referred to as a "healing-promoting method of the present disclosure"). Tissue, diseases, damage, a sheet-shaped cell culture used, and the like, which are targets in the healing-promoting method of the present disclosure, are as described in detail in the section describing the sheet-shaped cell culture of the present disclosure. In the healing-promoting method of the present disclosure, the sheet-shaped cell culture of the present disclosure or a component or the like can be used together with the components that enhance viability, engraftment and/or function of the sheet-shaped cell culture; the other active components and/or grafts useful for promoting healing of a target organ; and the like.

Another aspect of the present disclosure relates to the above-described method of regenerating tissue of a lumen organ having a damaged part on at least one side of a lumen wall in a target, the method including applying an effective amount of the sheet-shaped cell culture of the present disclosure or a composition or the like to a corresponding opposite site of the lumen wall of a site in which damage is present (hereinafter, sometimes referred to as a "regeneration method of the present disclosure"). Tissue, diseases, damage, a sheet-shaped cell culture used, and the like, which are targets in the regeneration method of the present disclosure, are as described in detail in the section describing the sheet-shaped cell culture of the present disclosure. In the regeneration method of the present disclosure, the sheet-shaped cell culture of the present disclosure or a component or the like can be used together with the components that enhance viability, engraftment and/or function of the sheet-shaped cell culture; the other active components and/or grafts useful for regenerating a target organ; and the like.

The healing-promoting method or the regeneration method of the present disclosure may further include a step of producing a sheet-shaped cell culture according to the production method of the present disclosure. The healing-promoting method or the regeneration method of the present disclosure may further include a step of collecting cells or a tissue serving as a source of cells for producing a sheet-shaped cell culture from a target before the step of producing a sheet-shaped cell culture. In one aspect, a target from which cells or tissue serving as the source of the cells is collected is the same individual as a target to which the sheet-shaped cell culture or a composition or the like is applied. In another aspect, a target from which cells or tissue serving as the source of the cells is collected is a different individual of the same species as a target to which the sheet-shaped cell culture or a composition or the like is applied. In still another aspect, a target from which cells or tissue serving as the source of the cells is collected is an individual of a different species from a target to which the sheet-shaped cell culture or a composition or the like is applied.

In the present disclosure, the term "target" means any living individual, preferably an animal, more preferably a mammal, and even more preferably a human individual.

In the present disclosure, an effective amount is, for example, an amount (for example, a size, a weight, the number of sheet-shaped cell cultures, and the like) which enables promoting of healing or regeneration of a target organ, and it is preferably an amount that enables healing or regeneration of the target organ. Such an amount can be appropriately determined by, for example, a test on an experimental animal such as mice, rats, dogs, or pigs, or a disease model animal, and such a test method is well known to those skilled in the art. In addition, a level of damage to an organ that is a target of promotion of healing or regeneration may be an important index for determining an effective amount.

As a method of application, in a case where there is damage on the inner portion of the lumen wall, application is typically performed to a corresponding outer portion of the lumen wall of such a damaged site by attachment. A frequency of application is typically once per treatment, but application of multiple times is possible in a case where a desired effect is not obtained.

Another aspect of the present disclosure relates to the above-described method for preventing perforation of a lumen organ in a target, the method including a step of applying the sheet-shaped cell culture of the present disclosure to the lumen organ (hereinafter, sometimes referred to as a "perforation prevention method of the present disclosure"). In the present disclosure, the term "perforation" particularly refers to, among penetrating damages, opening of a hole such that the contents of the lumen of the lumen organ are leaked, or a state in which a hole is open. In the present disclosure, the phrase "preventing perforation" means preventing perforation of a lumen organ at a site having the risk of developing the perforation. In the present disclosure, the phrase "site having the risk of developing the perforation" is a site in which a high probability of occurrence of perforation is medically predicted if no measure is taken, and examples thereof include, but are not limited to, a site damaged by surgery, a lesion area such as an ulcer or a tumor, and the like.

The perforation prevention method of the present disclosure is particularly suitably used for preventing postoperative perforation after a surgical treatment on a lumen organ since the risk of postoperative perforation at a treated site increases particularly when performing a surgical treatment on a lumen organ.

Examples of such surgical treatments include, but are not limited to, laparotomy, endoscopic surgery, and the like, but the surgical treatment is preferably endoscopic surgery, and especially a dissection procedure of a mucosa such as ESD and EMR, a mucous membrane, and/or a submucosal layer.

In the perforation prevention method of the present disclosure, the sheet-shaped cell culture is applied to a damaged part of the lumen wall or a corresponding opposite part of the damaged part. In one aspect, the damaged part is a damaged part generated at a site treated by a surgical treatment. Accordingly, in a preferable aspect of the perforation prevention method of the present disclosure, an application site is a site treated by a surgical treatment or a corresponding opposite site of the site treated by the surgical treatment.

In a case where the application site is the site treated by the surgical treatment or the corresponding opposite site of the site treated by the surgical treatment, penetrating damage may be generated at a procedure site during a surgical treatment, for example, a dissection procedure. That is, the perforation prevention method of the present disclosure can prevent postoperative perforation regardless of the presence or absence of penetrating damage occurring during a procedure, and for example, even in a case where penetrating damage occurs during a surgical treatment, it is possible to allow perforation to not to occur after the surgical treatment. Accordingly, in the perforation prevention method of the present disclosure, it is possible to prevent inflammation such as peritonitis caused by a surgical treatment such as ESD and postoperative perforation occurring after the procedure by applying the sheet-shaped cell culture.

In another aspect, the sheet-shaped cell culture is applied such that the sheet-shaped cell culture entirely covers a damaged part or a corresponding opposite part of the damaged part. Accordingly, as described above, one sheet-shaped cell culture larger than a damaged part, preferably a damaged part generated at a site that has been subjected to a surgical treatment, may be applied to cover the entire part, or a plurality of sheet-shaped cell cultures may be applied without gaps to cover the entire region.

In one aspect of the present disclosure, the sheet-shaped cell culture is delivered to the application site by a known delivery device or method for delivering the sheet-shaped cell culture. It is preferable that such devices or methods deliver the sheet-shaped cell culture in a less invasive manner. Various delivery devices have been developed to deliver fragile grafts such as, particularly the sheet-shaped cell culture, into a body, and such delivery devices can be used in the method of the present disclosure. For example, delivery devices disclosed in JP-A-2008-173333, JP-A-2009-511, and the like may be used, but the device is not limited thereto.

In one aspect, such a device has an operation unit, a long part, and an application part from the proximal side, in which the application part holds the sheet-shaped cell culture, the application part and the long part are inserted into a body to deliver the sheet-shaped cell culture, and thereby the sheet-shaped cell culture can be applied to the application site inside the body by operating the manual operation unit outside the body.

The perforation prevention method of the present disclosure may further include a step of producing a sheet-shaped cell culture according to the production method of the present disclosure. The perforation prevention method of the present disclosure may further include a step of collecting cells or a tissue serving as a source of cells for producing a sheet-shaped cell culture from a target before the step of producing a sheet-shaped cell culture. In one aspect, a target from which cells or tissue serving as the source of the cells is collected is the same individual as a target to which the sheet-shaped cell culture or a composition or the like is applied. In another aspect, a target from which cells or tissue serving as the source of the cells is collected is a different individual of the same species as a target to which the sheet-shaped cell culture or a composition or the like is applied. In still another aspect, a target from which cells or tissue serving as the source of the cells is collected is an individual of a different species from a target to which the sheet-shaped cell culture or a composition or the like is applied.

As mentioned above, ESD of duodenum has a high risk of developing intraoperative or postoperative complications, typically perforations, and the sheet-shaped cell cultures of the present disclosure can reduce the risk of developing such complications. Accordingly, one aspect of the present disclosure relates to the above-described method of reducing the risk of complications of endoscopic submucosal dissection of the duodenum, the method including applying the sheet-shaped cell culture of the present disclosure to an outer wall of the duodenum corresponding to a submucosal layer dissection site.

Another aspect of the present disclosure relates to a method for healing damage of a lumen organ in a target (hereinafter, sometimes referred to as a "damage-healing method of the present disclosure") . The damage-healing method of the present disclosure includes the following step:
step (A) of applying the sheet-shaped cell culture to a lumen organ.

The damage-healing method of the present disclosure may optionally include a step of providing the sheet-shaped cell culture of the present disclosure. In such a step, the sheet-shaped cell culture of the present disclosure is produced and provided. Such steps are as described in detail in the method for producing a sheet-shaped cell culture of the present disclosure.

In (A), the sheet-shaped cell culture of the present disclosure is applied to an application site of a lumen organ. In the damage-healing method of the present disclosure, the "application site" is a damaged part or a corresponding opposite part of the damaged part, as described above. The "lumen organ" is as described in detail above, but it is preferably an organ of the digestive system. Organs of the digestive system are also as described in detail above. At least one sheet-shaped cell culture is provided, but a plurality of, for example, two, three, four, or five sheets may be provided.

The damage is as described in detail above, and it may be penetrating damage or non-penetrating damage, but it is preferably non-penetrating damage. Among them, non-penetrating damage present on the lumen side, that is, damage present only on the inner side is particularly preferable from the viewpoint that it is difficult to treat this damage by the conventional methods.

The type of damage is also not particularly limited and is as described above, but an avulsed wound is preferable among the wounds. A particularly preferable wound is damage caused due to dissection of a part of the lumen wall, and typical examples thereof include an avulsed wound cause due to a dissection procedure such as ESD or EMR.

In another preferable aspect, the lumen organ is the duodenum, and the damage is penetrating damage. That is, one preferable aspect of the healing method of the present disclosure is a method of healing penetrating damage in a duodenum. As described above, a lumen wall of the duodenum is thin and therefore the risk of occurrence of perforation is high, and worsening of damage is likely to progress and treatment of damage is difficult due to a severe environment in which the duodenum is exposed to various digestive fluids, but it is possible to suitably treat penetrating damage in the duodenum according to the healing method of the present disclosure.

In such an aspect, penetrating damage may be penetrating damage that is generated by deterioration of non-penetrating damage such as an avulsed wound or a lesion such as an ulcer, or penetrating damage may be a lacuna caused during an procedure such as dissection. The healing method of the present aspect may optionally include a step of suturing penetrating damage before applying the sheet-shaped cell culture of the present disclosure to an application site.

In another aspect, the sheet-shaped cell culture is applied such that the sheet-shaped cell culture entirely covers the application site. As described above, at least one sheet-shaped cell culture is provided, but one large sheet-shaped cell culture may cover the entire application site, or a plurality of sheet-shaped cell cultures may be applied to cover the entire application site.

The sheet-shaped cell culture of the present disclosure has a reinforcement layer containing a biocompatible gel such as fibrin gel. The reinforcement layer may be formed after the production of the sheet-shaped cell culture, that is, before step (A), or may be formed after the application of the sheet-shaped cell culture, that is, after step (A). A method for forming a reinforcement layer containing a biocompatible gel is as described in detail above.

The method of the present aspect may further include a step (A') of applying other compositions and/or grafts which promote regeneration and/or healing before or after step (A) . The "other compositions and/or grafts which promote regeneration and/or healing" are as detailed above.

The step of applying such compositions and/or grafts may be performed before or after step (A) . In a case where it is performed before (A), the other compositions and/or grafts which promote regeneration and/or healing are applied directly to an application site, and the sheet-shaped cell culture of the present disclosure is applied thereon. In a case where it is performed after (A), the other compositions and/or grafts which promote regeneration and/or healing are applied on the applied sheet-shaped cell culture. In addition, the sheet-shaped cell culture may optionally have a reinforcement layer, and this case, the reinforcement layer may be formed before applying the other compositions and/or grafts which promote regeneration and/or healing, or the reinforcement layer may be formed after applying the other compositions and/or grafts which promote regeneration and/or healing by spraying a biocompatible gel from above the applied other compositions and/or grafts.

In a case where the sheet-shaped cell culture of the present disclosure is applied to an application site, it is delivered to the application site using a delivery device and/or a method known in the present technical field. The delivery device is as described in detail in the perforation prevention method.

Another aspect of the present disclosure relates to a method for improving a state of a lumen organ after a treatment in a surgical treatment of a lumen organ (hereinafter, sometimes referred to as an "improvement method of the present disclosure"). By applying the sheet-shaped cell culture of the present disclosure to a procedure site of a surgical treatment or a corresponding opposite site of the procedure site, a state of the lumen organ after underwent the surgical treatment can be improved as compared to a case in which no application is performed. In the present disclosure, the phrase "improving a state of a lumen organ after a surgical treatment" means that a state of a lumen organ resulting from the surgical treatment becomes favorable as compared to a case in which the method of the present disclosure is not used, and typical examples thereof include reducing the risk of complications at a procedure site after a surgical treatment, normalizing tissue at a procedure site, and the like. Examples of complications include, but are not limited to, adhesion of lumen organs, occurrence of perforations, and the like. Examples of normalization of tissue include regeneration of tissue at a procedure site, maintaining continuity of tissue at a procedure site, and the like.

Accordingly, one preferable aspect of the improvement method of the present disclosure relates to a method for preventing adhesion of a procedure site after a treatment in a surgical treatment of a lumen organ. In addition, another preferable aspect relates to a method for maintaining continuity of tissue a procedure site, for example, an ulcer base in a surgical treatment of a lumen organ.

In the improvement method of the present disclosure, details such as a sheet-shaped cell culture and an application method are as described in detail in each of the above aspects.

Another aspect of the present disclosure relates to a method for treating a diseased part on a surface of a lumen wall (hereinafter, sometimes referred to as a "disease treatment method of the present disclosure"). The disease treatment method of the present disclosure includes the following steps:
Step (A) of dissecting the diseased part on the surface of the lumen wall; and
Step (b) of applying the sheet-shaped cell culture to a dissected portion obtained in step (a) or a corresponding opposite portion of the dissected portion.

In (a), the diseased part on the surface of the lumen wall is dissected. In the present disclosure, the "surface of the lumen wall" refers to a portion exposed to the outside on one side of the lumen wall, and it typically means, for example, a mucosal layer portion in the digestive tract wall. A disease that can be treated by the disease treatment method of the present disclosure is not particularly limited as long as it is a disease that occurs on the surface of a lumen organ, and examples thereof include tumors such as cancer, ulcers, inflammation, and the like, of which tumors or ulcers are preferable from the viewpoint that they are physically removed as a suitable treatment method. In the present disclosure, the "tumor" includes benign tumor and malignant tumor (cancer, malignant neoplasm). Cancer includes epithelial malignant tumor (cancer) and non-epithelial malignant tumor (sarcoma). The lumen organ in the disease treatment method of the present disclosure is as described in detail in each of the above aspects. Accordingly, in a preferable aspect, the disease treated in the disease treatment method of the present disclosure is gastrointestinal cancer. In such an aspect, the cancer is preferably a cancer that stops in the mucosal layer of the inner wall of the digestive tract. In the present disclosure, the term "inflammation" means inflammation activated and caused by mixing of pancreatic juice and bile leaking from the lumen wall into the abdominal cavity with digestive fluid, and it typically includes peritonitis caused by gastrointestinal surgical treatments and post-procedure gastrointestinal perforation. Such inflammation is typically accompanied by an increase in the expression of inflammatory cytokines such as TNF-α, IL-1, and IL-6 in plasma that causes an inflammatory response such as the induction of C-reactive protein (CRP). Accordingly, the presence or absence of inflammation can be investigated by examining an increase or decrease.

A method known in the present technical field may be used for dissection of the diseased part. For example, in the case of a gastrointestinal cancer, a diseased part of the inner wall of the digestive tract, preferably a mucosal layer (that is, a mucosa, a muscularis mucosa, and/or a submucosal layer) is dissected and removed. Examples of methods for dissecting an inner wall of a digestive tract include, but are not limited to, a method of removing a disease by dissection, such as endoscopic submucosal dissection (ESD), endoscopic mucosal resection (EMR), and polypectomy. In a preferable aspect, dissection of the diseased part is performed by endoscopic submucosal dissection (ESD) . An avulsed wound resulting from dissection of the diseased part may be penetrating damage or non-penetrating damage, but it is preferably non-penetrating damage. In a case where the wound is penetrating damage, a step of suturing penetrating damage may further be included before (b).

In (b), the sheet-shaped cell culture of the present disclosure is applied to the dissected portion or a corresponding opposite portion of the dissected portion (hereinafter, sometimes referred to as an "application site" in the disease treatment method of the present disclosure). The sheet-shaped cell culture, the application method, and the like in the disease method of the present disclosure are as described in detail in each of the aspects of the present disclosure.

### EXAMPLES

The present disclosure will be described in more detail with reference to the following examples, but these merely show specific examples of the present disclosure and are not intended to limit the present disclosure.

### Example 1. Production of sheet-shaped cell culture

### (1) Sheet-shaped cell culture of porcine skeletal muscle myoblast cells

The striated muscle of the porcine lower limb was collected under general anesthesia, and the collected tissue was treated with an enzyme digestive solution containing collagenase and trypsin and dispersed in a single cell. The cells were cultured under the conditions of 37°C and 5% CO₂ in a MCDB131 medium containing 15% FBS until they reached confluent, and the cells were recovered. The recovered cells were seeded in a temperature-responsive culture dish of 60 mm (UpCell^{(R)}, manufactured by Cell Seed) in an amount of 2.2 × 10⁷ cells and cultured in a DMEM/F12 medium containing 20% FBS for 12 hours. Thereafter, the temperature was lowered to 20°C, and the sheet-shaped cell culture was detached from the culture dish and recovered. A reinforcement layer was formed on the sheet-shaped cell culture with fibrin gel.

The sheet-shaped cell cultures of skeletal muscle myoblast cells before and after formation of the reinforcement layer with fibrin gel were stained with hematoxylin and eosin for histological analysis (FIGS. 1A and 1B).

### (2) Sheet-shaped cell culture of porcine adipose-derived mesenchymal stem cells

A sheet-shaped cell culture of porcine adipose-derived mesenchymal stem cells was prepared using the method disclosed in Example 1-1 of WO2017/130802. The obtained sheet-shaped cell culture of porcine adipose-derived mesenchymal stem cells was stained with hematoxylin and eosin for histological analysis (FIG. 1C).

### Example 2. Creation of duodenal ESD pig model

Duodenal ESD was performed under general anesthesia on the pig from which the striated muscle was collected in Example 1 of (1), and was used as a sheet treatment group (n = 5). In addition, duodenal ESD was similarly performed on the control pigs under general anesthesia, and was used as a control group (n = 7). That is, as duodenal ESD, a part of the lumen surface of the duodenum of a pig was dissected. The sheet-shaped cell culture of skeletal muscle myoblast cells created in Example 1 of (1) was attached (applied) to the serosal surface on the outside of the duodenum at the site corresponding to the ESD procedure site (the dissected site on the lumen surface of the duodenum) of the pig in the sheet treatment group, such that the dissected site on the lumen surface was entirely covered. Furthermore, the dissected site was covered with a pedunculated omentum piece such that the dissected site on the lumen surface was entirely covered from above (outside) of the attached sheet-shaped cell culture of skeletal muscle myoblast cells. In the sheet treatment groups, two cases were subjected to laparotomy under general anesthesia on the 14th day after the procedure, and the rest was subjected thereto on the 3rd day after the procedure to observe the duodenal ESD procedure site, and thereafter, the tissue was excised. In the control group, the sheet-shaped cell culture of skeletal muscle myoblast cells was not attached to the outer surface of the lumen organ at the dissected site on the lumen surface of the duodenum, and the control group was covered only with a pedunculated omentum. Among the control group, one case was subjected to laparotomy under general anesthesia on the 1st day after the procedure, another case was subjected thereto on the 2nd day after the procedure, and the rest was subjected thereto on the 3rd day after the procedure to observe the duodenal ESD procedure site, and thereafter, the tissue was excised. The presence or absence of perforated peritonitis, adhesion score, and histological healing status were compared to those of the control group.

The results are shown in the following table below and FIGS. 3 to 6.

**[Table 1]**

| | | Diameter of ulcer | Perforation during procedure | Sheet (cm/number) | Cover with omentum | Perforation after procedure | Excision time | Adhesion score |
|---|---|---|---|---|---|---|---|---|
| Control group | 1 | 1.5 | None | - | Covered | Perforation | 3 days after procedure | 2 |
| | 2 | 1.5 | Major | - | Covered | Perforation | 1 day after procedure | 2 |
| | 3 | 1.0 | Major | - | Covered | None^{*1} | 3 days after procedure | 3 |
| | 4 | 1.5 | None | - | Covered | Perforation | 2 days after procedure | 3 |
| | 5 | 1.5 | None | - | Covered | Perforation | 3 days after procedure | 2 |
| | 6 | 1.5 | Micro | - | Covered | Micro | 3 days after procedure | 2 |
| | 7 | 1.5 | Micro | - | Covered | Perforation | 3 days after procedure | 3 |
| Sheet treatment group | 1 | 1.5 | Micro | 1.5/2 | Covered | None | 3 days after procedure | 0 |
| | 2 | 1.5 | Micro | 2.5/1 | Covered | None | 3 days after procedure | 1 |
| | 3 | 1.5 | Micro | 2.8/1 | Covered | None | 3 days after procedure | 1 |
| | 4 | 1.5 | Major | 2.5/2 | Covered | None | 14 days after procedure | 3 |
| | 5 | 2 | None | 2.5/1 | Covered | None | 14 days after procedure | 3 |

| | | | | | | | | |
|---|---|---|---|---|---|---|---|---|
| The term "micro" indicates small perforation such that air leakage is observed, and the term "major" indicates perforation such that leakage of contents is observed *1: Tumor necrosis was observed Adhesion score 0: No adhesion, 1: Slight adhesion, 2: Adhesion requiring blunt dissection, 3: Adhesion requiring blunt dissection | | | | | | | | |

In the control group 1, although no perforation of ESD was observed during the operation, postoperative perforation could be confirmed at the stage confirmed on the third day after the procedure, and leakage of bile was recognized. In addition, adhesion with the surrounding small intestine was also recognized. It was also confirmed that continuity of the ulcer base disappeared in the histological stained image of the ESD procedure site (FIG. 3A).

In the control group 2, perforation of ESD was observed during the procedure, but because the condition after the procedure was poor, the procedure site was confirmed, and the tissue was excised on the first day after the procedure. Perforation was confirmed in the procedure site, and bile leakage and adhesion with the surrounding small intestine were also recognized. It was also confirmed that continuity of the ulcer base disappeared in the histological stained image of the ESD procedure site (FIG. 3B).

In the sheet treatment group 1, air leakage from the procedure site was recognized during the ESD procedure, and it was considered that there was slight perforation. Two sheets of skeletal muscle myoblast cells having a diameter of 1.5 cm and reinforced with fibrin gel were attached to the serosa side of the site corresponding to the procedure site, and then covered with the omentum. When it was checked on the third day after the procedure, neither perforation nor adhesion was recognized. It was confirmed that the continuity of the ulcer base was maintained even in the histological stained image (FIGS. 4A and 4B).

In the sheet treatment group 2, air leakage from the procedure site was recognized during the ESD procedure, and it was considered that there was slight perforation. One sheet of skeletal muscle myoblast cells having a diameter of 2.5 cm and reinforced with fibrin gel was attached to the serosal side of the site corresponding to the procedure site, and then covered with omentum. When it was checked 3 days after the procedure, no perforation was recognized, but a blunt dissectable adhesion with the surrounding small intestine was recognized. It was confirmed that the continuity of the ulcer base was maintained even in the histological stained image (FIGS. 4C and 4D).

In the sheet treatment group 3, air leakage from the procedure site (a site indicated by the arrow in FIG. 3E) was recognized during the ESD procedure, and it was considered that there was slight perforation. One sheet of skeletal muscle myoblast cells having a diameter of 2.8 cm and reinforced with fibrin gel was attached to the serosa side of the site corresponding to the procedure site, and then covered with the omentum. When it was checked on the third day after the procedure, neither perforation nor adhesion was recognized. The histological stained image also confirmed that the continuity of the ulcer base was maintained, the migration of inflammatory cells was confirmed by hematoxylin-eosin (HE) staining, and the engraftment of the sheet of myoblast cells was confirmed by desmin staining (FIGS. 4E and 4F).

In the sheet treatment group 4, air leakage from the procedure site (a site indicated by the arrow in FIGS. 4G and 4H) was recognized during the ESD procedure, and it was considered that there was slight perforation. Two sheets of skeletal muscle myoblast cells having a diameter of 2.5 cm and reinforced with fibrin gel were attached to the serosa side of the site corresponding to the procedure site, and then covered with the omentum. When it was checked 14 days after the procedure, collagen fiber hyperplasia and wall thickening were recognized, and the ulcer was scarred. Accordingly, it was understood that, by attaching the sheet and clogging the hole, the cells were attracted to the part of the tissue that had become thinner due to ESD, thereby making the part thicker, and the sheet of myoblast cells was engrafted by hematoxylin and eosin (HE) staining and azan staining (FIGS. 4G and 4H).

In the sheet treatment group 5, air leakage from the procedure site was not recognized during the ESD procedure, and it was considered that there was no perforation. One sheet of skeletal muscle myoblast cells having a diameter of 2.5 cm and reinforced with fibrin gel was attached to the serosal side of the site corresponding to the procedure site, and then covered with omentum. When it was checked 14 days after the procedure, collagen fiber hyperplasia and wall thickening were recognized, and the ulcer was scarred. Accordingly, it was understood that, by attaching the sheet and clogging the hole, the cells were attracted to the part of the tissue that had become thinner due to ESD, thereby making the part thicker.

The presence or absence of perforated peritonitis (inflammatory reaction) was examined as follows.

Transition of inflammatory reaction was measured by an increase or decrease in expression of C-reactive protein (CRP) and TNF-α.

Specifically, pig blood was collected at the time of transplantation (day 0), 1 day after transplantation (day 1), and 3 days after transplantation (day 3), and serum was obtained by centrifugal separation. Concentrations of CRP and TNF-α contained in these sera were measured with a commercially available CRP and TNF-α measurement ELISA kit. The results are shown in FIG. 6. As shown in FIG. 6, it can be seen that concentrations of CRP and TNF-α, which are inflammatorymarkers, were lower in the sheet-transplanted group than in the control group.

Accordingly, in the control group, there was an open hole and a leaked out liquid which caused inflammation, whereas in the sheet treatment group, the attachment of the sheet could prevent inflammation due to liquid leakage (FIG. 6).

### Reference Signs List

- 11: MUCOSA
- 12: SUBMUCOSAL LAYER (BRUNNER'S GLAND)
- 20: MUSCULAR LAYER

## Claims

1. A sheet-shaped cell culture for promoting healing of a lumen organ having a damaged part on at least one side of a lumen wall, wherein the sheet-shaped cell culture is applied to a corresponding opposite site of the lumen wall of a site in which damage is present.

2. The sheet-shaped cell culture according to claim 1, wherein the damage is on an inner portion of the lumen wall, and the sheet-shaped cell culture is applied to a corresponding outer portion of the lumen wall, or the damage is on an outer portion of the lumen wall, and the sheet-shaped cell culture is applied to a corresponding inner portion of the lumen wall.

3. The sheet-shaped cell culture according to claim 1, wherein the damage is penetrating damage or non-penetrating damage.

4. The sheet-shaped cell culture according to any one of claims 1 to 3, wherein the lumen organ is an organ of a digestive system.

5. The sheet-shaped cell culture according to any one of claims 1 to 4, wherein the damage is caused by dissection of surface tissue of the lumen wall.

6. The sheet-shaped cell culture according to any one of claims 1 to 5, comprising a reinforcement layer containing a gel and/or a polymer.

7. The sheet-shaped cell culture according to any one of claims 1 to 6, wherein the sheet-shaped cell culture is applied together with a pedunculated blood vessel.

8. The sheet-shaped cell culture according to any one of claims 1 to 7, wherein the sheet-shaped cell culture has a thickness of 50 to 500 µm.

9. The sheet-shaped cell culture according to any one of claims 1 to 8, wherein the sheet-shaped cell culture contains a cell population having a seeding density of about 7.1 × 10⁵ cells/cm² to about 3.0 × 10⁶ cells/cm².

10. The sheet-shaped cell culture according to any one of claims 1 to 9, wherein the sheet-shaped cell culture is for preventing inflammation occurring due to a surgical treatment and/or inflammation occurring due to perforation caused by a surgical treatment.

11. A method for preventing perforation in a lumen organ after a surgical treatment, the method comprising:
a step of applying a sheet-shaped cell culture to the lumen organ,
wherein the sheet-shaped cell culture is applied to a treated site or a corresponding opposite site of the treated site.

12. The method according to claim 11, wherein the surgical treatment is dissection of a mucosa, a muscularis mucosa, and/or a submucosal layer of the lumen organ.

13. The method according to claim 11 or 12, wherein the sheet-shaped cell culture is applied such that the sheet-shaped cell culture entirely covers the treated site or the corresponding opposite site of the treated site.

14. The method according to any one of claims 11 to 13, wherein the sheet-shaped cell culture is delivered to the treated site or the corresponding opposite site of the treated site by a delivery device.

15. A method for treating a diseased part on a surface of a lumen wall, the method comprising:
step (a) of dissecting the diseased part on the surface of the lumen wall; and
step (b) of applying the sheet-shaped cell culture to a dissected portion obtained in step (a) or a corresponding opposite portion of the dissected portion.

16. The method according to claim 15, wherein a disease is cancer.

17. The method according to claim 15 or 16, wherein the dissection of the diseased part on the surface of the lumen wall is dissection of a mucosa, a muscularis mucosa, and/or a submucosal layer having the diseased part.
